# EUROPEAN PATENT SPECIFICATION

(11) **EP 3 390 632 B1**
(45) Date of publication and mention of the grant of the patent: **10.09.2025**
(21) Application number: 16825944.8
(22) Date of filing: 15.12.2016
(51) Int. Cl.: C12N 15/11, C12N 15/113, C12N 15/10, C12N 15/81

(54) **METHODS AND COMPOSITIONS FOR POLYMERASE II (POL-II) BASED GUIDE RNA EXPRESSION**
VERFAHREN UND ZUSAMMENSETZUNGEN FÜR POLYMERASE II (POL-II)-BASIERTE GUIDE-RNA-EXPRESSION
PROCÉDÉS ET COMPOSITIONS POUR L'EXPRESSION D'ARN GUIDE BASÉE SUR LA POLYMÉRASE II (POL-II)

(30) Priority: 18.12.2015 US 201562269122 P
(43) Date of publication of application: 24.10.2018
(73) Proprietor: Danisco US Inc., Palo Alto, California 94304 (US)
(72) Inventor: BENDEZU, Felipe Oseas, Palo Alto California 94304 (US); FAN, Xiaochun, Palo Alto California 94304 (US); FRISCH,Ryan L., Palo Alto California 94304 (US); HONG, Seung-Pyo, Palo Alto,CA 94304 (US)
(74) Representative: Dehns
(86) International application number: PCT/US2016/066772
(87) International publication number: WO 2017/106414

(56) References cited:
- WO-A1-2013/176772
- WO-A1-2015/099850
- WO-A1-2015/138855
- WO-A1-2015/153940
- WO-A1-2016/110511
- WO-A2-2015/048707
- SHIN YOSHIOKA ET AL: "Development of a mono-promoter-driven CRISPR/Cas9 system in mammalian cells", SCIENTIFIC REPORTS, vol. 5, 16 December 2015 (2015-12-16), pages 18341, XP055282633, DOI: 10.1038/srep18341
- YANGBIN GAO ET AL: "Self-processing of ribozyme-flanked RNAs into guide RNAs in vitro and in vivo for CRISPR-mediated genome editing", JOURNAL OF INTEGRATIVE PLANT BIOLOGY, vol. 56, no. 4, 6 April 2014 (2014-04-06), pages 343 - 349, XP055175728, ISSN: 1672-9072, DOI: 10.1111/jipb.12152
- GAO SHULIANG ET AL: "Multiplex gene editing of theYarrowia lipolyticagenome using the CRISPR-Cas9 system", JOURNAL OF INDUSTRIAL MICROBIOLOGY AND BIOTECHNOLOGY, BASINGSTOKE, GB, vol. 43, no. 8, 27 June 2016 (2016-06-27), pages 1085 - 1093, XP036000917, ISSN: 1367-5435, [retrieved on 20160627], DOI: 10.1007/S10295-016-1789-8

## Description

### FIELD

The disclosure relates to the field of molecular biology, in particular, to methods for producing guide RNAs and methods for altering the genome of a cell.

### BACKGROUND

Recombinant DNA technology has made it possible to insert DNA sequences at targeted genomic locations and/or modify (edit) specific endogenous chromosomal sequences, thus altering the organism's phenotype. Site-specific integration techniques, which employ site-specific recombination systems, as well as other types of recombination technologies, have been used to generate targeted insertions of genes of interest in a variety of organism. Genome-editing techniques such as designer zinc finger nucleases (ZFNs), transcription activator-like effector nucleases (TALENs), homing meganucleases, engineered nucleases are available for producing targeted genome perturbations, but these systems tends to have a low specificity and employ designed nucleases that need to be redesigned for each target site, which renders them costly and time-consuming to prepare. CRISPR-associated (Cas) RNA-guided endonuclease systems have been developed as a means for introducing site-specific DNA strand breaks at specific target sites These nuclease based systems can create a single strand or double strand break (DSB) in a target nucleotide, which can increase the frequency of homologous recombination at the target locus.

Inhibition of gene expression can be accomplished, for example, by interrupting or deleting the DNA sequence of the gene, resulting in "knock-out" of the gene. Gene knock-outs mostly have been carried out through homologous recombination (HR), a technique applicable across a wide array of organisms from bacteria to mammals. Another tool for studying gene function can be through genetic "knock-in", which is also usually performed by HR. HR for purposes of gene targeting (knock-out or knock-in) can use the presence of an exogenously supplied DNA having homology with the target site. Although gene targeting by HR is a powerful tool, it can be a complex, labor-intensive procedure. Most studies using HR have generally been limited to knock-out of a single gene rather than multiple genes in a pathway, since HR is generally difficult to scale-up in a cost-effective manner. This difficulty is exacerbated in organisms in which HR is not efficient. Such low efficiency typically forces practitioners to rely on selectable phenotypes or exogenous markers to help identify cells in which a desired HR event occurred.

WO 2015/138855 describes expression vectors containing a nucleic acid encoding an RNA polymerase III promoter, a self-cleaving ribozyme, a CRISPR-Cas9 single guide RNA and an RNA polymerase III terminator, wherein the ribozyme is 5' to the CRISPR-Cas9 single guide RNA. Vectors encoding a single guide RNA (either with or without a 5' ribozyme) under the control of an RNA polymerase II promoter are also disclosed.

Gao & Zhao (Journal of Integrative Plant Biology 56(4): 343-349, 2014) describes DNA constructs comprising single guide RNA genes flanked by self-cleaving ribozymes and under the control of RNA polymerase-II promoters.

WO 2015/153940 describes two types of DNA constructs comprising polynucleotides encoding a single guide RNA under the control of an RNA polymerase-II promoter. These constructs comprise either Csy4 recognition sites or self-cleaving ribozymes flanking the sgRNA gene.

WO 2015/099850 describes DNA constructs which comprise an RNA polymerase-II promoter which drives expression of a polynucleotide which encodes a single guide RNA flanked by Csy4 recognition sites.

There remains a need for new and more efficient genome engineering technologies that are affordable, easy to set up, scalable, and amenable to targeting multiple positions within the genome of an organism.

### BRIEF SUMMARY

Compositions and methods are provided for editing nucleotides and/or altering target sites in the genome of a cell. In general terms the methods and compositions employ a recombinant DNA construct comprising a Pol-II promoter operably linked to a polynucleotide encoding a single guide RNA, wherein said guide RNA is capable of forming a guide RNA/Cas endonuclease complex, wherein said complex can bind to and cleave a target site sequence in the genome of a cell such as a microbial cell, as specified below. The present disclosure further describes methods and compositions employing said recombinant DNA construct to modify the genome of non-conventional yeast.

In a first aspect, provided herein is a recombinant DNA construct comprising:
(i) a Polymerase II (Pol-II) promoter fused to its 5' UTR and operably linked to a polynucleotide encoding a single guide RNA comprising a variable targeting domain and a Cas endonuclease recognition domain;
(ii) a 3' processing element operably linked to the polynucleotide; and, optionally,
(iii) a terminator located 3' of said processing element;
wherein said guide RNA is capable of forming a guide RNA/Cas endonuclease complex, which complex can bind to and cleave a target site sequence in the genome of a eukaryote, and wherein said polynucleotide contains no 5' processing element..

In a second aspect, provided herein is a non-human eukaryote comprising the recombinant DNA construct of the first aspect. In an embodiment the non-human eukaryote is a non-conventional yeast. The non-conventional yeast can be a member of a genus selected from the group consisting of *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces,* and *Pachysolen.*

In a third aspect, provided herein is an expression vector comprising at least one recombinant DNA construct of the first aspect.

In a fourth aspect, provided herein is a method for modifying a target site on a chromosome or episome in a non-conventional yeast, the method comprising providing to a non-conventional yeast at least a first recombinant DNA construct of the first aspect and a second recombinant DNA construct encoding a Cas endonuclease, wherein the Cas endonuclease introduces a single or double-strand break at said target site; and
wherein said method is not performed for the treatment of a human or animal body.

In a fifth aspect, provided herein is a method for editing a nucleotide sequence on a chromosome or episome in a non-conventional yeast, the method comprising providing to a non-conventional yeast a polynucleotide modification template DNA, a first recombinant DNA construct comprising a DNA sequence encoding a Cas endonuclease, and a second recombinant DNA construct of the first aspect, wherein the Cas endonuclease introduces a single or double-strand break at a target site in the chromosome or episome of said yeast, wherein said polynucleotide modification template DNA comprises at least one nucleotide modification of said nucleotide sequence; and
wherein said method is not performed for the treatment of a human or animal body.

In an sixth aspect, provided herein is a method for silencing a nucleotide sequence on a chromosome or episome in a non-conventional yeast, the method comprising providing to a non-conventional yeast at least a first recombinant DNA construct comprising a DNA sequence encoding an inactivated Cas endonuclease, and at least a second recombinant DNA construct of the first aspect, wherein the guide RNA molecule and the inactivated Cas endonuclease can form a complex that binds to said nucleotide sequence in the chromosome or episome of said yeast, thereby blocking transcription of said nucleotide sequence; and
wherein said method is not performed for the treatment of a human or animal body.

Additional embodiments of the methods and compositions provided herein are described below.

### BRIEF DESCRIPTION OF THE DRAWINGS AND THE SEQUENCE LISTING

The disclosure can be more fully understood from the following detailed description and the accompanying drawings and Sequence Listing.

### Figures

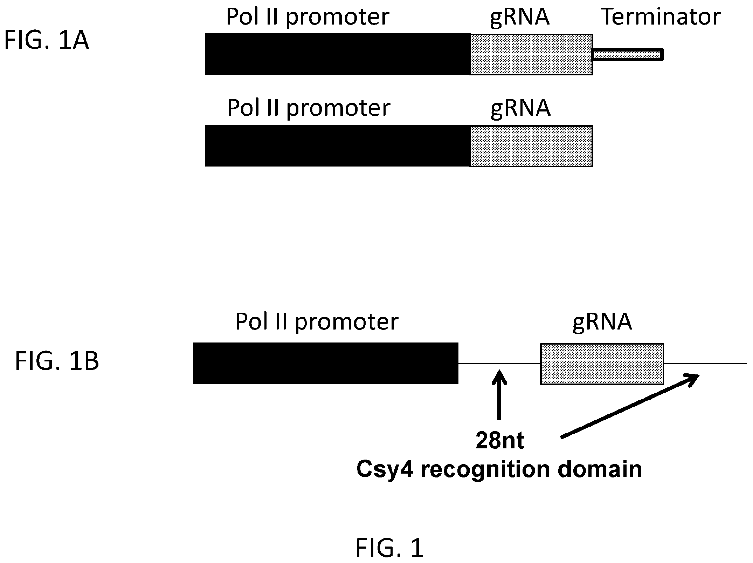

**Figure 1A-1B** show guide RNA expression cassettes. **FIG. 1A** shows a Pol-II promoter operably linked to a DNA encoding a guide RNA expression cassette with and without a terminator. **FIG. 1B** shows a Pol-II promoter operably linked to DNA encoding a Csy4 recognition domain, a DNA encoding a guide RNA expression cassettes and a DNA encoding a Csy4 recognition domain.

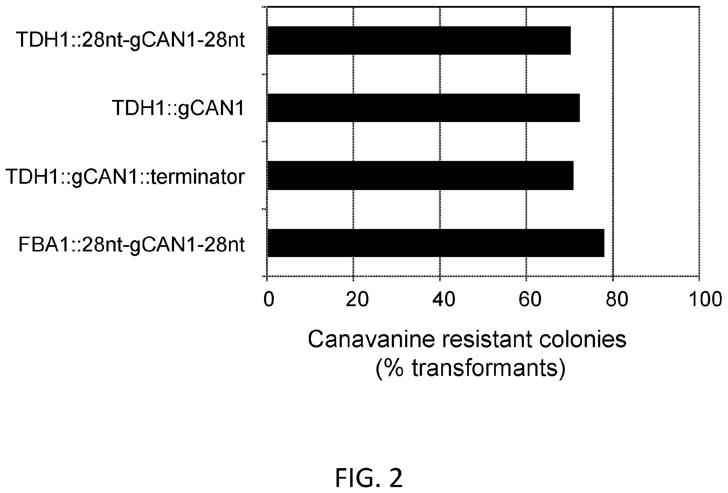

**Figure 2** shows *can1* mutation frequencies from different gRNA expression plasmid transformants. The *can1* mutation frequencies were calculated by comparing the number of colonies on canavanine plates (CanR) and the total number of transformants on CM-ura plates. TDH1::28nt-gCAN1-28nt (pYRH376), TDH1::gCAN1 (pYRH378), TDH1::gCAN1::terminator (pYRH379), and FBA1::28nt-gCAN1-28nt (pYRH380)

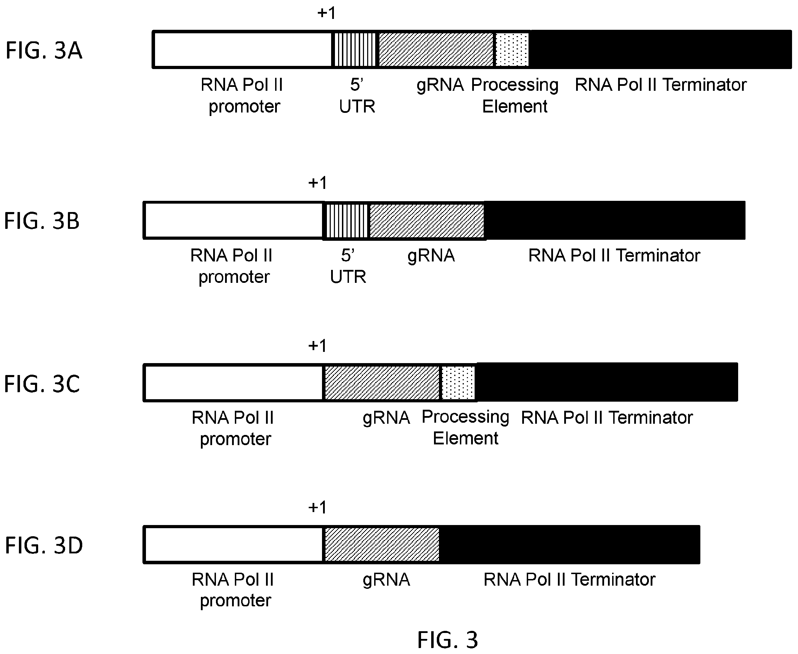

**Figure 3A-3D** are diagram showing four different RNA polymerase II (Pol-II) gRNA expression cassettes. White filled box represents the RNA polymerase II promoter, the DNA encoding the gRNA (diagonal stripe fill) can either be fused to the end of the 5' untranslated region (vertical stripe fill) or the 5' end of the promoter (transcriptional start site. The 3' end of the DNA encoding the gRNA can be fused directly the the RNA polymerase III transcriptional terminator (Black fill) or to a processing element (dot fill) (eg. HDV ribozyme).

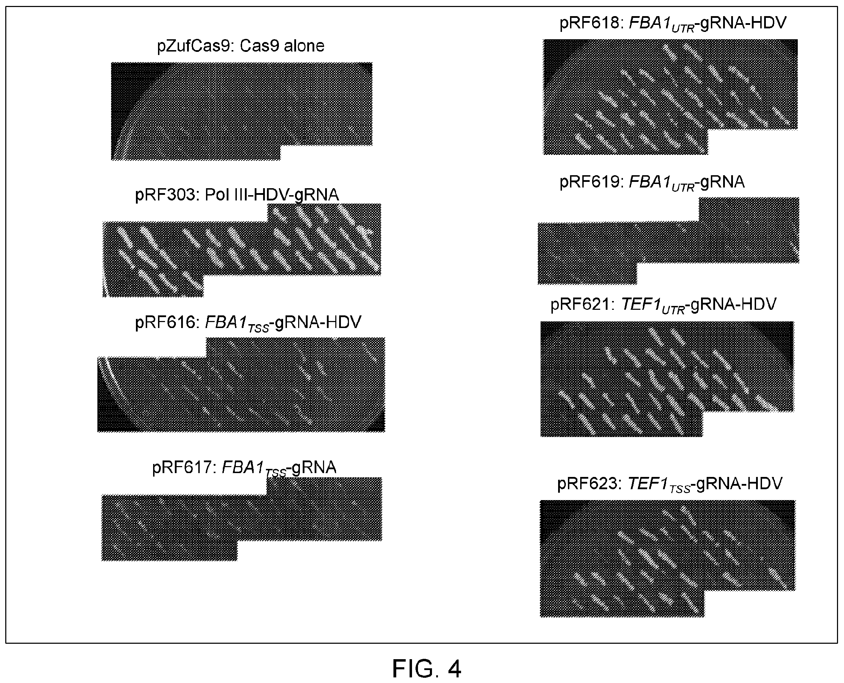

**Figure 4****:** presents images of L-Canavanine containing agar plates containing patches of *Y. lipolytica* primary transformants with different DNA constructs. Colony growth indicates loss of function of the *CAN1* gene.

### Sequences

**Table 1**

| **Summary of Nucleic Acid and Protein SEQ ID Numbers** | | |
|---|---|---|
| Description | Nucleic acid SEQ ID NO. | Protein SEQ ID NO. |
| Cas9 endonuclease, *Streptococcus pyogenes* | 1 | |
| FBA1 promoter | 2 | |
| Yarrowia codon optimized P. aeruginosa Csy4 | 3 | |
| TDH1:28bp-gCAN1-28bp | 4 | |
| Csy4 recognition sequence | 5 | |
| Csy4 recognition sequence flanked sgRNA | 6 | |
| sgRNA targeted sequence of CAN1 | 7 | |
| TDH1 promoter | 8 | |
| NLS fused to Yarrowia codon optimized P. aeruginosa Csy4 | 9 | |
| Simian virus 40 (SV40) monopartite amino terminal nuclear localization signal | 10 | |
| ARS18 sequence | 11 | |
| full length ARS18 sequence | 12 | |
| FBA1 terminator | 13 | |
| Yarrowia codon optimized Cas9 | 14 | |
| SV40 Nuclear localization signal | | 15 |
| FBA1 promoter | 16 | |
| Yarrowia optimized expression cassette | 17 | |
| pZufCas9 | 18 | |
| TEF1tss promoter fragment | 19 | |
| tef1 promoter forward | 20 | |
| tef1tss promoter reverse | 21 | |
| TEF1UTR promoter fragment | 22 | |
| Tef1utr promoter reverse | 23 | |
| FBA1tss promoter fragment | 24 | |
| FBA1 promoter forward | 25 | |
| FBA1tss reverse | 26 | |
| FBA1utr promoter fragment | 27 | |
| FBA1utr reverse | 28 | |
| ACT1 for gRNA | 29 | |
| pFB23 | 30 | |
| Act1 CER forward | 31 | |
| ACT1 reverse | 32 | |
| ACT1 for HDV gRNA | 33 | |
| ACT1 HDV forward | 34 | |
| Can1-1 for FBA1tss | 35 | |
| pRF84; | 36 | |
| Can1-1 FBA1tss forward | 37 | |
| Can1-1 ACT1 reverse | 38 | |
| Can1-1-HDV for FBA1tss | 39 | |
| Can1-1-HDV Act 1 reverse | 40 | |
| Can1-1 for FBA1utr | 41 | |
| Can1-1 FBA1utr forward | 42 | |
| Can1-1-HDV for FBA1utr | 43 | |
| Can1-1 for TEF1tss; | 44 | |
| Can1-1 for TEF1tss forward | 45 | |
| Can1-1-HDV for TEF1tss | 46 | |
| Can1-1 for TEF1utr | 47 | |
| Can1-1 TEF1utr forward | 48 | |
| Can1-1-HDV for Tef1utr | 49 | |
| FBA1TSS-Can1-1-ACT1 cassette | 50 | |
| FBA1TSS-Can1-1HDV-ACT1 cassette | 51 | |
| FBA1UTR-Can1-1-ACT1 cassette | 52 | |
| FBA1UTR-Can1-1HDV-ACT1 cassette | 53 | |
| TEF1TSS-Can1-1-ACT1 cassette | 54 | |
| TEF1TSS-Can1-1HDV-ACT1 cassette | 55 | |
| TEF1UTR-Can1-1-ACT1 Cassette | 56 | |
| TEF1UTR-Can1-1HDV-ACT1 cassette | 57 | |
| HY009 | 58 | |
| HY010 | 59 | |
| ON476 | 60 | |
| pRF617 | 61 | |
| pRF616 | 62 | |
| pRF619 | 63 | |
| pRF618 | 64 | |
| pRF626 | 65 | |
| pRF625 | 66 | |
| pRF623 | 67 | |
| pRF621 | 68 | |
| Can1-1 target site | 69 | |
| pRF303 | 70 | |

### DETAILED DESCRIPTION

Compositions and methods are provided for editing nucleotides and/or altering target sites in the genome of a cell. The methods and compositions employ a recombinant DNA construct comprising:
(i) a Polymerase II (Pol-II) promoter fused to its 5' UTR and operably linked to a polynucleotide encoding a single guide RNA comprising a variable targeting domain and a Cas endonuclease recognition domain;
(ii) a 3' processing element operably linked to the polynucleotide; and, optionally,
(iii) a terminator located 3' of said processing element;
wherein said guide RNA is capable of forming a guide RNA/Cas endonuclease complex, which complex can bind to and cleave a target site sequence in the genome of a eukaryote, and wherein said polynucleotide contains no 5' processing element.

CRISPR (clustered regularly interspaced short palindromic repeats) loci refers to certain genetic loci encoding factors of class I, II, or III DNA cleavage systems, for example, used by bacterial and archaeal cells to destroy foreign DNA (Horvath and Barrangou, 2010, Science 327:167-170). Components of CRISPR systems are taken advantage of herein in a heterologous manner for DNA targeting in cells.

The type II CRISPR/Cas system from bacteria employs a crRNA (CRISPR RNA) and tracrRNA (trans-activating CRISPR RNA) to guide the Cas endonuclease to its DNA target. The crRNA contains a region complementary to one strand of the double strand DNA target and a region that base pairs with the tracrRNA (trans-activating CRISPR RNA) forming a RNA duplex that directs the Cas endonuclease to cleave the DNA target. CRISPR systems belong to different classes, with different repeat patterns, sets of genes, and species ranges. The number of CRISPR-associated genes at a given CRISPR locus can vary between species (Haft et al. (2005) Computational Biology, PLoS Comput Biol 1(6): e60. doi:10.1371/journal.pcbi.0010060).

The term "Cas gene" herein refers to a gene that is generally coupled, associated or close to, or in the vicinity of flanking CRISPR loci. The terms "Cas gene", "CRISPR-associated (Cas) gene" are used interchangeably herein. The term "Cas endonuclease" herein refers to a protein encoded by a Cas gene. A Cas endonuclease herein, when in complex with a suitable polynucleotide component, is capable of recognizing, binding to, and optionally nicking or cleaving all or part of a specific DNA target sequence. A Cas endonuclease described herein comprises one or more nuclease domains. Cas endonucleases of the disclosure include those having a HNH or HNH-like nuclease domain and / or a RuvC or RuvC-like nuclease domain. A Cas endonuclease of the disclosure includes a Cas9 protein, a Cpf1 protein, a C2c1 protein, a C2c2 protein, a C2c3 protein, Cas3, Cas 5, Cas7, Cas8, Cas10, or complexes of these.

As used herein, the terms "guide polynucleotide/Cas endonuclease complex", "guide polynucleotide/Cas endonuclease system", "guide polynucleotide/Cas complex", "guide polynucleotide/Cas system", "guided Cas system" are used interchangeably herein and refer to at least one guide polynucleotide and at least one Cas endonuclease that are capable of forming a complex, wherein said guide polynucleotide/Cas endonuclease complex can direct the Cas endonuclease to a DNA target site, enabling the Cas endonuclease to recognize, bind to, and optionally nick or cleave (introduce a single or double strand break into) the DNA target site. A guide polynucleotide/Cas endonuclease complex herein can comprise Cas protein(s) and suitable polynucleotide component(s) of any of the four known CRISPR systems (Horvath and Barrangou, 2010, Science 327:167-170) such as a type I, II, or III CRISPR system. A Cas endonuclease unwinds the DNA duplex at the target sequence and optionally cleaves at least one DNA strand, as mediated by recognition of the target sequence by a polynucleotide (such as, but not limited to, a crRNA or guide RNA) that is in complex with the Cas protein. Such recognition and cutting of a target sequence by a Cas endonuclease typically occurs if the correct protospaceradjacent motif (PAM) is located at or adjacent to the 3' end of the DNA target sequence. Alternatively, a Cas protein herein may lack DNA cleavage or nicking activity, but can still specifically bind to a DNA target sequence when complexed with a suitable RNA component. (See also U.S. Patent Application US 2015/0082478, published on March 19, 2015 and US 2015/0059010, published on February 26, 2015.)

A guide polynucleotide/Cas endonuclease complex can cleave one or both strands of a DNA target sequence. A guide polynucleotide/Cas endonuclease complex that can cleave both strands of a DNA target sequence typically comprises a Cas protein that has all of its endonuclease domains in a functional state (e.g., wild type endonuclease domains or variants thereof retaining some or all activity in each endonuclease domain). Thus, a wild type Cas protein (e.g., a Cas9 protein disclosed herein), or a variant thereof retaining some or all activity in each endonuclease domain of the Cas protein, is a suitable example of a Cas endonuclease that can cleave both strands of a DNA target sequence. A Cas9 protein comprising functional RuvC and HNH nuclease domains is an example of a Cas protein that can cleave both strands of a DNA target sequence. A guide polynucleotide/Cas endonuclease complex that can cleave one strand of a DNA target sequence can be characterized herein as having nickase activity (e.g., partial cleaving capability). A Cas nickase typically comprises one functional endonuclease domain that allows the Cas to cleave only one strand (i.e., make a nick) of a DNA target sequence. For example, a Cas9 nickase may comprise (i) a mutant, dysfunctional RuvC domain and (ii) a functional HNH domain (e.g., wild type HNH domain). As another example, a Cas9 nickase may comprise (i) a functional RuvC domain (e.g., wild type RuvC domain) and (ii) a mutant, dysfunctional HNH domain. Non-limiting examples of Cas9 nickases suitable for use herein are disclosed in U.S. Patent Appl. Publ. No. 2014/0189896.

A pair of Cas9 nickases can be used to increase the specificity of DNA targeting. In general, this can be done by providing two Cas9 nickases that, by virtue of being associated with RNA components with different guide sequences, target and nick nearby DNA sequences on opposite strands in the region for desired targeting. Such nearby cleavage of each DNA strand creates a double strand break (i.e., a DSB with single-stranded overhangs), which is then recognized as a substrate for non-homologous-end-joining, NHEJ (prone to imperfect repair leading to mutations) or homologous recombination, HR. Each nick in these embodiments can be at least about 5, 10, 15, 20, 30, 40, 50, 60, 70, 80, 90, or 100 (or any integer between 5 and 100) bases apart from each other, for example. One or two Cas9 nickase proteins herein can be used in a Cas9 nickase pair. For example, a Cas9 nickase with a mutant RuvC domain, but functioning HNH domain (i.e., Cas9 HNH+/RuvC-), could be used (e.g., *Streptococcus pyogenes* Cas9 HNH+/RuvC-). Each Cas9 nickase (e.g., Cas9 HNH+/RuvC-) would be directed to specific DNA sites nearby each other (up to 100 base pairs apart) by using suitable RNA components herein with guide RNA sequences targeting each nickase to each specific DNA site.

A Cas protein can be part of a fusion protein comprising one or more heterologous protein domains (e.g., 1, 2, 3, or more domains in addition to the Cas protein). Such a fusion protein may comprise any additional protein sequence, and optionally a linker sequence between any two domains, such as between Cas and a first heterologous domain. Examples of protein domains that may be fused to a Cas protein herein include, without limitation, epitope tags (e.g., histidine [His], V5, FLAG, influenza hemagglutinin [HA], myc, VSV-G, thioredoxin [Trx]), reporters (e.g., glutathione-5-transferase [GST], horseradish peroxidase [HRP], chloramphenicol acetyltransferase [CAT], beta-galactosidase, beta-glucuronidase [GUS], luciferase, green fluorescent protein [GFP], HcRed, DsRed, cyan fluorescent protein [CFP], yellow fluorescent protein [YFP], blue fluorescent protein [BFP]), and domains having one or more of the following activities: methylase activity, demethylase activity, transcription activation activity (e.g., VP16 or VP64), transcription repression activity, transcription release factor activity, histone modification activity, RNA cleavage activity and nucleic acid binding activity. A Cas protein can also be in fusion with a protein that binds DNA molecules or other molecules, such as maltose binding protein (MBP), S-tag, Lex A DNA binding domain (DBD), GAL4A DNA binding domain, and herpes simplex virus (HSV) VP16.

A Cas protein herein can be from any of the following genera: *Aeropyrum, Pyrobaculum, Sulfolobus, Archaeoglobus, Haloarcula, Methanobacteriumn, Methanococcus, Methanosarcina, Methanopyrus, Pyrococcus, Picrophilus, Thernioplasnia, Corynebacterium, Mycobacterium, Streptomyces, Aquifrx, Porphvromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myrococcus, Campylobacter, Wolinella, Acinetobacter, Erwinia, Escherichia, Legionella, Methylococcus, Pasteurella, Photobacterium, Salmonella, Xanthomonas, Yersinia, Streptococcus, Treponema, Francisella,* or *Thermotoga.* See also WO 2016/186946 for more examples of Cas proteins.

A guide polynucleotide/Cas endonuclease complex in certain embodiments can bind to a DNA target site sequence, but does not cleave any strand at the target site sequence. Such a complex may comprise a Cas protein in which all of its nuclease domains are mutant, dysfunctional. For example, a Cas9 protein herein that can bind to a DNA target site sequence, but does not cleave any strand at the target site sequence, may comprise both a mutant, dysfunctional RuvC domain and a mutant, dysfunctional HNH domain. A Cas protein herein that binds, but does not cleave, a target DNA sequence can be used to modulate gene expression, for example, in which case the Cas protein could be fused with a transcription factor (or portion thereof) (e.g., a repressor or activator, such as any of those disclosed herein).

The Cas endonuclease gene herein can encode a Type II Cas9 endonuclease, such as but not limited to, Cas9 genes listed in SEQ ID NOs: 462, 474, 489, 494, 499, 505, and 518 of WO 2007/025097, published March 1, 2007. In another embodiment, the Cas endonuclease gene is a microbe or optimized Cas9 endonuclease gene. The Cas endonuclease gene can be operably linked to a SV40 nuclear targeting signal upstream of the Cas codon region and a bipartite VirD2 nuclear localization signal (Tinland et al. (1992) Proc. Natl. Acad. Sci. USA 89:7442-6) downstream of the Cas codon region.

The Cas endonuclease gene includes a plant or microbial codon optimized *Streptococcus pyogenes* Cas9 gene that can recognize any genomic sequence of the form N(12-30)NGG and can in principle be targeted or a Cas9 endonuclease originated from an organism selected from the group consisting of *Brevibacillus laterosporus, Lactobacillus reuteri* Mlc3, *Lactobacillus rossiae* DSM 15814, *Pediococcus pentosaceus* SL4, *Lactobacillus nodensis* JCM 14932, *Sulfurospirillum* sp. SCADC, *Bifidobacterium thermophilum* DSM 20210, *Loktanella vestfoldensis, Sphingomonas sanxanigenens* NX02, *Epilithonimonas tenax* DSM 16811, *Sporocytophaga myxococcoides* and *Psychroflexus torquis* ATCC 700755, wherein said Cas9 endonuclease can form a guide RNA/Cas endonuclease complex capable of recognizing, binding to, and optionally nicking or cleaving all or part of a DNA target sequence. Other Cas endonuclease systems have been described in WO 2016/186946.

"Cas9" (formerly referred to as Cas5, Csn1, or Csx12) herein refers to a Cas endonuclease of a type II CRISPR system that forms a complex with a crNucleotide and a tracrNucleotide, or with a single guide polynucleotide, for specifically recognizing and cleaving all or part of a DNA target sequence. Cas9 protein comprises a RuvC nuclease domain and an HNH (H-N-H) nuclease domain, each of which can cleave a single DNA strand at a target sequence (the concerted action of both domains leads to DNA double-strand cleavage, whereas activity of one domain leads to a nick). In general, the RuvC domain comprises subdomains I, II and III, where domain I is located near the N-terminus of Cas9 and subdomains II and III are located in the middle of the protein, flanking the HNH domain (Hsu et al, Cell 157:1262-1278). A type II CRISPR system includes a DNA cleavage system utilizing a Cas9 endonuclease in complex with at least one polynucleotide component. For example, a Cas9 can be in complex with a CRISPR RNA (crRNA) and a trans-activating CRISPR RNA (tracrRNA). In another example, a Cas9 can be in complex with a single guide RNA.

The amino acid sequence of a Cas9 protein described herein, as well as certain other Cas proteins herein, may be derived from a *Streptococcus* (e.g., *S. pyogenes, S. pneumoniae, S. thermophilus, S. agalactiae, S. parasanguinis, S. oralis, S. salivarius,* S. macacae, *S. dysgalactiae, S. anginosus, S. constellatus, S. pseudoporcinus, S. mutans), Listeria* (e.g., *L. innocua), Spiroplasma* (e.g., S. *apis,* S. *syrphidicola), Peptostreptococcaceae, Atopobium, Porphyromonas* (e.g., *P. catoniae), Prevotella* (e.g., *P. intermedia), Veillonella, Treponema* (e.g., *T. socranskii, T. denticola), Capnocytophaga, Finegoldia* (e.g., *F. magna), Coriobacteriaceae* (e.g., C. *bacterium), Olsenella* (e.g., *O. profusa), Haemophilus* (e.g., *H. sputorum, H. pittmaniae), Pasteurella* (e.g., *P. bettyae), Olivibacter* (e.g., *O. sitiensis), Epilithonimonas* (e.g., *E. tenax), Mesonia* (e.g., *M. mobilis), Lactobacillus* (e.g., L. *plantarum), Bacillus* (e.g., *B. cereus), Aquimarina* (e.g., *A. muelleri), Chryseobacterium* (e.g., *C. palustre), Bacteroides* (e.g., *B. graminisolvens), Neisseria* (e.g., *N. meningitidis), Francisella* (e.g., *F. novicida),* or *Flavobacterium* (e.g., F. *frigidarium, F. soli)* species, for example. As another example, a Cas9 protein can be any of the Cas9 proteins disclosed in Chylinski et al. (RNA Biology 10:726-737 and WO 2016/186946).

Accordingly, the sequence of a Cas9 protein herein can comprise, for example, any of the Cas9 amino acid sequences disclosed in GenBank Accession Nos. G3ECR1 *(S. thermophilus),* WP_026709422, WP_027202655, WP_027318179, WP_027347504, WP_027376815, WP_027414302, WP_027821588, WP_027886314, WP_027963583, WP_028123848, WP_028298935, Q03JI6 (*S*. *thermophilus),* EGP66723, EGS38969, EGV05092, EHI65578 (*S*. *pseudoporcinus),* EIC75614 (*S*. *oralis),* EID22027 (*S*. *constellatus),* EIJ69711, EJP22331 (*S. oralis*)*,* EJP26004 (*S*. *anginosus),* EJP30321, EPZ44001 *(S. pyogenes),* EPZ46028 (*S*. *pyogenes),* EQL78043 (*S*. *pyogenes),* EQL78548 (*S*. *pyogenes),* ERL10511, ERL12345, ERL19088 (*S*. *pyogenes),* ESA57807 (*S*. *pyogenes),* ESA59254 (*S*. *pyogenes*)*,* ESU85303 (*S*. *pyogenes),* ETS96804, UC75522, EGR87316 (*S*. *dysgalactiae*)*,* EGS33732, EGV01468 (*S*. *oralis*)*,* EHJ52063 (S. *macacae*)*,* EID26207 (*S. oralis*)*,* EID33364, EIG27013 (*S. parasanguinis*)*,* EJF37476, EJO19166 (*Streptococcus* sp. BS35b), EJU16049, EJU32481, YP_006298249, ERF61304, ERK04546, ETJ95568 (*S*. *agalactiae*)*,* TS89875, ETS90967 (*Streptococcus* sp. SR4), ETS92439, EUB27844 (*Streptococcus* sp. BS21), AFJ08616, EUC82735 (*Streptococcus* sp. CM6), EWC92088, EWC94390, EJP25691, YP_008027038, YP_008868573, AGM26527, AHK22391, AHB36273, Q927P4, G3ECR1, or Q99ZW2 *(S. pyogenes).* A variant of any of these Cas9 protein sequences may be used, but should have specific binding activity, and optionally endonucleolytic activity, toward DNA when associated with an RNA component herein. Such a variant may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence of the reference Cas9.

Alternatively, a Cas9 protein may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to any of the foregoing amino acid sequences, for example. Such a variant Cas9 protein should have specific binding activity, and optionally cleavage or nicking activity, toward DNA when associated with an RNA component herein.

A Cas protein herein such as a Cas9 can comprise a heterologous nuclear localization sequence (NLS). A heterologous NLS amino acid sequence herein may be of sufficient strength to drive accumulation of a Cas protein in a detectable amount in the nucleus of a yeast cell herein, for example. An NLS may comprise one (monopartite) or more (e.g., bipartite) short sequences (e.g., 2 to 20 residues) of basic, positively charged residues (e.g., lysine and/or arginine), and can be located anywhere in a Cas amino acid sequence but such that it is exposed on the protein surface. An NLS may be operably linked to the N-terminus or C-terminus of a Cas protein herein, for example. Two or more NLS sequences can be linked to a Cas protein, for example, such as on both the N- and C-termini of a Cas protein. Non-limiting examples of suitable NLS sequences herein include those disclosed in U.S. Patent No. 7,309,576.

The Cas endonuclease can comprise a modified form of the Cas9 polypeptide. The modified form of the Cas9 polypeptide can include an amino acid change (e.g., deletion, insertion, or substitution) that reduces the naturally-occurring nuclease activity of the Cas9 protein. For example, in some instances, the modified form of the Cas9 protein has less than 50%, less than 40%, less than 30%, less than 20%, less than 10%, less than 5%, or less than 1% of the nuclease activity of the corresponding wild-type Cas9 polypeptide (US patent application US 2014/0068797, published on March 6, 2014). In some cases, the modified form of the Cas9 polypeptide has no substantial nuclease activity and is referred to as catalytically "inactivated Cas9" or "deactivated cas9 (dCas9)." Catalytically inactivated Cas9 variants include Cas9 variants that contain mutations in the HNH and RuvC nuclease domains. These catalytically inactivated Cas9 variants are capable of interacting with sgRNA and binding to the target site in vivo but cannot cleave either strand of the target DNA.

A catalytically inactive Cas9 can be fused to a heterologous sequence (US patent application US 2014/0068797, published on March 6, 2014). Suitable fusion partners include, but are not limited to, a polypeptide that provides an activity that indirectly increases transcription by acting directly on the target DNA or on a polypeptide (e.g., a histone or other DNA-binding protein) associated with the target DNA. Additional suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity. Further suitable fusion partners include, but are not limited to, a polypeptide that directly provides for increased transcription of the target nucleic acid (e.g., a transcription activator or a fragment thereof, a protein or fragment thereof that recruits a transcription activator, a small molecule/drug-responsive transcription regulator, etc.). A catalytically inactive Cas9 can also be fused to a FokI nuclease to generate double strand breaks (Guilinger et al. Nature biotechnology, volume 32, number 6, June 2014).

The terms "functional fragment ", "fragment that is functionally equivalent" and "functionally equivalent fragment" of a Cas endonuclease are used interchangeably herein, and refer to a portion or subsequence of the Cas endonuclease sequence of the present disclosure in which the ability to recognize, bind to, and optionally nick or cleave (introduce a single or double strand break in) the target site is retained.

The terms "functional variant", "Variant that is functionally equivalent" and "functionally equivalent variant" of a Cas endonuclease are used interchangeably herein, and refer to a variant of the Cas endonuclease of the present disclosure in which the ability to recognize, bind to, and optionally nick or cleave (introduce a single or double strand break in) the target site is retained. Fragments and variants can be obtained via methods such as site-directed mutagenesis and synthetic construction.

Any guided endonuclease can be used in the methods disclosed herein. Such endonucleases include, but are not limited to Cas9 and Cpf1 endonucleases. Many endonucleases have been described to date that can recognize specific PAM sequences (see for example Jinek et al. (2012) Science 337 p 816-821, WO 2016/186946 and Zetsche B et al. 2015. Cell 163, 1013) and cleave the target DNA at a specific position. It is understood that based on the methods and embodiments described herein utilizing a guided Cas system one can now tailor these methods such that they can utilize any guided endonuclease system.

The term "off-target site effects" and "off-target effects" are used interchangeably and include any alteration in an off-target site that is due to the activity of an endonuclease cleavage, wherein the alteration include, for example: (i) a replacement of at least one nucleotide, (ii) a deletion of at least one nucleotide, (iii) an insertion of at least one nucleotide, or (iv) any combination of (i) - (iii), as well as any integration of a template or donor DNA at an unintended site. The unintended site can be any site in the genome of the organism that is not the target site.

Several approaches have been explored to improve the specificity and decrease off-target site effects of Cas endonucleases, including reducing the amount of enzyme active in the cell, shortening the section of the guide RNA complementary to the target, deploying pairs of engineered nicking Cas9s (Nicolas et al. Human Gene Therapy. 2015, 26(7): 425-431), and structure-guided protein engineering (Slaymaker et al. Science. 2015. Science DOI: 10.1126/science.aad5227). Many of these approaches remain to have limitations, often decreasing on-target editing efficiency.

The endonuclease can be provided to a cell by any method known in the art, for example, but not limited to transient introduction methods, transfection, microinjection, and/or topical application or indirectly via recombination constructs. The endonuclease can be provided as a protein or as a guided polynucleotide complex directly to a cell or indirectly via recombination constructs. The endonuclease can be introduced into a cell transiently or can be incorporated into the genome of the host cell using any method known in the art. Uptake of the endonuclease and/or the guided polynucleotide into the cell can be facilitated with a Cell Penetrating Peptide (CPP) as described in WO 2016/073433.

Endonucleases are enzymes that cleave the phosphodiester bond within a polynucleotide chain, and include restriction endonucleases that cleave DNA at specific sites without damaging the bases. Restriction endonucleases include Type I, Type II, Type III, and Type IV endonucleases, which further include subtypes. In the Type I and Type III systems, both the methylase and restriction activities are contained in a single complex. Endonucleases also include meganucleases, also known as homing endonucleases (HEases), which like restriction endonucleases, bind and cut at a specific recognition site, however the recognition sites for meganucleases are typically longer, about 18 bp or more (WO 2012/129373). Meganucleases have been classified into four families based on conserved sequence motifs, the families are the LAGLIDADG, GIY-YIG, H-N-H, and His-Cys box families. These motifs participate in the coordination of metal ions and hydrolysis of phosphodiester bonds. HEases are notable for their long recognition sites, and for tolerating some sequence polymorphisms in their DNA substrates. The naming convention for meganuclease is similar to the convention for other restriction endonuclease. Meganucleases are also characterized by prefix F-, I-, or PI- for enzymes encoded by free-standing ORFs, introns, and inteins, respectively. One step in the recombination process involves polynucleotide cleavage at or near the recognition site. This cleaving activity can be used to produce a double-strand break. For reviews of site-specific recombinases and their recognition sites, see, Sauer (1994) Curr Op Biotechnol 5:521-7; and Sadowski (1993) FASEB 7:760-7. In some examples the recombinase is from the Integrase or Resolvase families.

TAL effector nucleases (TALEN) are a class of sequence-specific nucleases that can be used to make double-strand breaks at specific target sequences in the genome of a plant or other organism. (Miller et al. (2011) Nature Biotechnology 29:143-148). Zinc finger nucleases (ZFNs) are engineered double-strand break inducing agents comprised of a zinc finger DNA binding domain and a double-strand-break-inducing agent domain. Recognition site specificity is conferred by the zinc finger domain, which typically comprising two, three, or four zinc fingers, for example having a C2H2 structure, however other zinc finger structures are known and have been engineered. Zinc finger domains are amenable for designing polypeptides which specifically bind a selected polynucleotide recognition sequence. ZFNs include an engineered DNA-binding zinc finger domain linked to a non-specific endonuclease domain, for example nuclease domain from a Type IIs endonuclease such as Fokl. Additional functionalities can be fused to the zinc-finger binding domain, including transcriptional activator domains, transcription repressor domains, and methylases. In some examples, dimerization of nuclease domain is required for cleavage activity. Each zinc finger recognizes three consecutive base pairs in the target DNA. For example, a 3 finger domain recognized a sequence of 9 contiguous nucleotides, with a dimerization requirement of the nuclease, two sets of zinc finger triplets are used to bind an 18 nucleotide recognition sequence.

As used herein, the term "guide polynucleotide", relates to a polynucleotide sequence that can form a complex with a Cas endonuclease and enables the Cas endonuclease to recognize, bind to, and optionally cleave a DNA target site. The guide polynucleotide referred to herein is a single molecule and is a RNA sequence. Optionally, the guide polynucleotide can comprise at least one nucleotide, phosphodiester bond or linkage modification such as, but not limited, to Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide RNA" or "gRNA" (See also U.S. Patent Application US 2015/0082478, published on March 19, 2015 and US 2015/0059010, published on February 26, 2015).

The guide polynucleotide (also referred to as single guide polynucleotide) comprises a crNucleotide sequence linked to a tracrNucleotide sequence. The single guide polynucleotide comprises a first nucleotide sequence domain (referred to as Variable Targeting domain or VT domain) that can hybridize to a nucleotide sequence in a target DNA and a Cas endonuclease recognition domain (CER domain), that interacts with a Cas endonuclease polypeptide. By "domain" it is meant a contiguous stretch of RNA nucleotides . Thus, the VT domain and /or the CER domain of a single guide polynucleotide comprises a RNA sequence. The single guide polynucleotide being comprised of sequences from the crNucleotide and the tracrNucleotide may be referred to as "single guide RNA" (which is composed of a contiguous stretch of RNA nucleotides). The single guide polynucleotide can form a complex with a Cas endonuclease, wherein said guide polynucleotide/Cas endonuclease complex (also referred to as a guide polynucleotide/Cas endonuclease system) can direct the Cas endonuclease to a genomic target site, enabling the Cas endonuclease to recognize, bind to, and optionally nick or cleave (introduce a single or double strand break) the target site. (See also U.S. Patent Application US 2015/0082478, published on March 19, 2015 and US 2015/0059010, published on February 26, 2015.)

The term "variable targeting domain" or "VT domain" is used interchangeably herein and includes a nucleotide sequence that can hybridize (is complementary) to one strand (nucleotide sequence) of a double strand DNA target site. The % complementation between the first nucleotide sequence domain (VT domain ) and the target sequence can be at least 50%, 51%, 52%, 53%, 54%, 55%, 56%, 57%, 58%, 59%, 60%, 61%, 62%, 63%, 63%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The variable targeting domain can be at least 12, 13, 14, 15, 16, 17, 18, 19, 20, 21, 22, 23, 24, 25, 26, 27, 28, 29 or 30 nucleotides in length. In some embodiments, the variable targeting domain comprises a contiguous stretch of 12 to 30 nucleotides. The variable targeting domain can be composed of a RNA sequence, a modified RNA sequence, or any combination thereof.

The term "Cas endonuclease recognition domain" or "CER domain" (of a guide polynucleotide) is used interchangeably herein and includes a nucleotide sequence that interacts with a Cas endonuclease polypeptide. A CER domain comprises a tracrNucleotide mate sequence followed by a tracrNucleotide sequence. The CER domain can be composed of a RNA sequence, a modified RNA sequence (see for example US 2015/0059010, published on February 26, 2015), or any combination thereof.

The nucleotide sequence linking the crNucleotide and the tracrNucleotide of a single guide polynucleotide comprises a RNA sequence. In one embodiment, the nucleotide sequence linking the crNucleotide and the tracrNucleotide of a single guide polynucleotide can be at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99 or 100 nucleotides in length. In another embodiment, the nucleotide sequence linking the crNucleotide and the tracrNucleotide of a single guide polynucleotide can comprise a tetraloop sequence, such as, but not limited to a GAAA tetraloop sequence.

Nucleotide sequence modification of the guide polynucleotide, VT domain and/or CER domain can be selected from, but not limited to , the group consisting of a 5' cap, a 3' polyadenylated tail, a riboswitch sequence, a stability control sequence, a sequence that forms a dsRNA duplex, a modification or sequence that targets the guide poly nucleotide to a subcellular location, a modification or sequence that provides for tracking , a modification or sequence that provides a binding site for proteins , a Locked Nucleic Acid (LNA), a 5-methyl dC nucleotide, a 2,6-Diaminopurine nucleotide, a 2'-Fluoro A nucleotide, a 2'-Fluoro U nucleotide; a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, wherein the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

The terms "functional fragment ", "fragment that is functionally equivalent" and "functionally equivalent fragment" of a guide RNA, crRNA or tracrRNA are used interchangeably herein, and refer to a portion or subsequence of the guide RNA crRNA or tracrRNA, respectively, of the present disclosure in which the ability to function as a guide RNA, crRNA or tracrRNA, respectively, is retained.

The terms "functional variant ", "Variant that is functionally equivalent" and "functionally equivalent variant" of a guide RNA, crRNA or tracrRNA (respectively) are used interchangeably herein, and refer to a variant of the guide RNA, crRNA or tracrRNA, respectively, of the present disclosure in which the ability to function as a guide RNA, crRNA or tracrRNA, respectively, is retained.

The terms "single guide RNA" and "sgRNA" are used interchangeably herein and relate to a synthetic fusion of two RNA molecules, a crRNA (CRISPR RNA) comprising a variable targeting domain (linked to a tracr mate sequence that hybridizes to a tracrRNA), fused to a tracrRNA (trans-activating CRISPR RNA). The single guide RNA can comprise a crRNA or crRNA fragment and a tracrRNA or tracrRNA fragment of the type II CRISPR/Cas system that can form a complex with a type II Cas endonuclease, wherein said guide RNA/Cas endonuclease complex can direct the Cas endonuclease to a DNA target site, enabling the Cas endonuclease to recognize, bind to, and optionally nick or cleave (introduce a single or double strand break) the DNA target site.

There remains a need for improved expression systems of guide RNAs in cells. Described herein are compositions and methods using Pol-II pormoters to express guide RNAs capable of guiding a Cas endonuclease to its target site.
In one embodiment, provided here in is a recombinant DNA construct comprising:
(i) a Polymerase II (Pol-II) promoter fused to its 5' UTR and operably linked to a polynucleotide encoding a single guide RNA comprising a variable targeting domain and a Cas endonuclease recognition domain;
(ii) a 3' processing element operably linked to the polynucleotide; and, optionally,
(iii) a terminator located 3' of said processing element;
wherein said guide RNA is capable of forming a guide RNA/Cas endonuclease complex, which complex can bind to and cleave a target site sequence in the genome of a eukaryote, and wherein said polynucleotide contains no 5' processing element.

The terms "guide RNA/Cas endonuclease complex", "guide RNA/Cas endonuclease system", " guide RNA/Cas complex", "guide RNA/Cas system", "gRNA/Cas complex", "gRNA/Cas system", "RNA-guided endonuclease" , "RGEN" are used interchangeably herein and refer to at least one RNA component and at least one Cas endonuclease that are capable of forming a complex, wherein said guide RNA/Cas endonuclease complex can direct the Cas endonuclease to a DNA target site, enabling the Cas endonuclease to recognize, bind to, and optionally nick or cleave (introduce a single or double strand break) the DNA target site. A guide RNA/Cas endonuclease complex herein can comprise Cas protein(s) and suitable RNA component(s) of any of the four known CRISPR systems (Horvath and Barrangou, 2010, Science 327:167-170) such as a type I, II, or III CRISPR system. A guide RNA/Cas endonuclease complex can comprise a Type II Cas9 endonuclease and a gRNA). (See also U.S. Patent Application US 2015/0082478, published on March 19, 2015 and US 2015/0059010, published on February 26, 2015.)

The guide polynucleotide can be introduced into a cell transiently, as single stranded polynucleotide or a double stranded polynucleotide, using any method known in the art such as, but not limited to, particle bombardment, *Agrobacterium transformation* or topical applications. The guide polynucleotide can also be introduced indirectly into a cell by introducing a recombinant DNA molecule (via methods such as, but not limited to, particle bombardment or *Agrobacterium transformation*) comprising a heterologous nucleic acid fragment encoding a guide polynucleotide, operably linked to a specific promoter that is capable of transcribing the guide RNA in said cell.

A RNA polymerase III promoter (Pol-III promoter) can allow for transcription of RNA with precisely defined, unmodified, 5'- and 3'-ends (DiCarlo et al., Nucleic Acids Res. 41: 4336-4343; Ma et al., Mol. Ther. Nucleic Acids 3:e161; SNR52 promoter, Marck et al. 2006. Nuceic Acid Res. 34(6):1816-1835)

RNA polymerase II (RNAP II and Pol-II) is an enzyme found in eukaryotic cells. It catalyzes the transcription of DNA to synthesize precursors of mRNA and most snRNA and microRNA (Kornberg R (1999). "Eukaryotic transcriptional control". Trends in Cell Biology 9 (12): M46. doi:10.1016/S0962-8924(99)01679-7. PMID 10611681; Sims, R. J. 3rd; Mandal, S. S.; Reinberg, D. (June 2004). "Recent highlights of RNA-polymerase-II-mediated transcription". Current opinion in cell biology 16 (3): 263-271.doi:10.1016/j.ceb.2004.04.004. ISSN 0955-0674.

RNA Polymerase II promoters are well known in the art (for review see Butler J. and Kadonaga J. 2002. The RNA polymerase II core promoter: a key component in the regulation of gene expression. GENES & DEVELOPMENT 16:2583-2592). RNA polymerase II promoters include the *FBA1* promoter (Hong et al. 2012. Yeast. 29:59-72).

The terms "Pol-II guide RNA expression cassette" and "Polymerase II-gRNA expression cassette" are used interchangeable used herein and refer to an expression cassette (recombinant DNA construct) wherein a Polymerase II promoter encodes a guide RNA.

The terms "target site", "target sequence", "target site sequence, "target DNA", "target locus", "genomic target site", "genomic target sequence", "genomic target locus" and "protospacer", are used interchangeably herein and refer to a polynucleotide sequence such as, but not limited to, a nucleotide sequence on a chromosome, episome, or any other DNA molecule in the genome (including chromosomal, choloroplastic, mitochondrial DNA, plasmid DNA) of a cell, at which a guide polynucleotide/Cas endonuclease complex can recognize, bind to, and optionally nick or cleave . The target site can be an endogenous site in the genome of a cell, or alternatively, the target site can be heterologous to the cell and thereby not be naturally occurring in the genome of the cell, or the target site can be found in a heterologous genomic location compared to where it occurs in nature. As used herein, terms "endogenous target sequence" and "native target sequence" are used interchangeable herein to refer to a target sequence that is endogenous or native to the genome of a cell and is at the endogenous or native position of that target sequence in the genome of the cell. Cells include, but are not limited to, human, non-human, animal, bacterial, fungal, insect, yeast, non-conventional yeast, and plant cells as well as plants and seeds produced by the methods described herein. An "artificial target site" or "artificial target sequence" are used interchangeably herein and refer to a target sequence that has been introduced into the genome of a cell. Such an artificial target sequence can be identical in sequence to an endogenous or native target sequence in the genome of a cell but be located in a different position (*i.e*., a nonendogenous or non-native position) in the genome of a cell.

An "altered target site", "altered target sequence", "modified target site", "modified target sequence" are used interchangeably herein and refer to a target sequence as disclosed herein that comprises at least one alteration when compared to non-altered target sequence. Such "alterations" include, for example: (i) replacement of at least one nucleotide, (ii) a deletion of at least one nucleotide, (iii) an insertion of at least one nucleotide, or (iv) any combination of (i) - (iii).

Methods for "modifying a target site" and for "altering a target site" are used interchangeably herein and refer to methods for producing an altered target site.

The length of the target DNA sequence (target site) can vary, and includes, for example, target sites that are at least 12, 13, 14, 15, 16, 17, 18, 19, 20, 21, 22, 23, 24, 25, 26, 27, 28, 29, 30 or more nucleotides in length. It is further possible that the target site can be palindromic, that is, the sequence on one strand reads the same in the opposite direction on the complementary strand. The nick/cleavage site can be within the target sequence or the nick/cleavage site could be outside of the target sequence. In another variation, the cleavage could occur at nucleotide positions immediately opposite each other to produce a blunt end cut or, in other Cases, the incisions could be staggered to produce single-stranded overhangs, also called "sticky ends", which can be either 5' overhangs, or 3' overhangs. Active variants of genomic target sites can also be used. Such active variants can comprise at least 65%, 70%, 75%, 80%, 85%, 90%, 91%, 92%, 93%, 94%, 95%, 96%, 97%, 98%, 99% or more sequence identity to the given target site, wherein the active variants retain biological activity and hence are capable of being recognized and cleaved by an Cas endonuclease. Assays to measure the single or double-strand break of a target site by an endonuclease are known in the art and generally measure the overall activity and specificity of the agent on DNA substrates containing recognition sites.

A "protospacer adjacent motif" (PAM) herein refers to a short nucleotide sequence adjacent to a target sequence (protospacer) that is recognized (targeted) by a guide polynucleotide/Cas endonuclease system described herein. The Cas endonuclease may not successfully recognize a target DNA sequence if the target DNA sequence is not followed by a PAM sequence. The sequence and length of a PAM herein can differ depending on the Cas protein or Cas protein complex used. The PAM sequence can be of any length but is typically 0, 1, 2, 3, 4, 5, 6, 7, 8, 9, 10, 11, 12, 13, 14, 15, 16, 17, 18, 19 or 20 nucleotides long.

The terms "targeting", "gene targeting" and "DNA targeting" are used interchangeably herein. DNA targeting herein may be the specific introduction of a knock-out, edit, or knock-in at a particular DNA sequence, such as in a chromosome or plasmid of a cell. In general, DNA targeting can be performed herein by cleaving one or both strands at a specific DNA sequence in a cell with an endonuclease associated with a suitable polynucleotide component. Such DNA cleavage, if a double-strand break (DSB), can prompt NHEJ or HDR processes which can lead to modifications at the target site.

A targeting method herein can be performed in such a way that two or more DNA target sites are targeted in the method, for example. Such a method can optionally be characterized as a multiplex method. Two, three, four, five, six, seven, eight, nine, ten, or more target sites can be targeted at the same time in certain embodiments. A multiplex method is typically performed by a targeting method herein in which multiple different RNA components are provided, each designed to guide a guide polynucleotide/Cas endonuclease complex to a unique DNA target site.

The terms "knock-out", "gene knock-out" and "genetic knock-out" are used interchangeably herein. A knock-out represents a DNA sequence of a cell that has been rendered partially or completely inoperative by targeting with a Cas protein; such a DNA sequence prior to knock-out could have encoded an amino acid sequence, or could have had a regulatory function (e.g., promoter), for example. A knock-out may be produced by an indel (insertion or deletion of nucleotide bases in a target DNA sequence through NHEJ), or by specific removal of sequence that reduces or completely destroys the function of sequence at or near the targeting site.

The guide polynucleotide/Cas endonuclease system can be used in combination with a co-delivered polynucleotide modification template to allow for editing (modification) of a genomic nucleotide sequence of interest. (See also U.S. Patent Application US 2015/0082478, published on March 19, 2015 and WO 2015/026886, published on February 26, 2015.)

A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, or (iv) any combination of (i) - (iii).

The term "polynucleotide modification template" includes a polynucleotide that comprises at least one nucleotide modification when compared to the nucleotide sequence to be edited. A nucleotide modification can be at least one nucleotide substitution, addition or deletion. Optionally, the polynucleotide modification template can further comprise homologous nucleotide sequences flanking the at least one nucleotide modification, wherein the flanking homologous nucleotide sequences provide sufficient homology to the desired nucleotide sequence to be edited.

Genome editing can be accomplished using any method of gene editing available. For example, gene editing can be accomplished through the introduction into a host cell of a polynucleotide modification template (sometimes also referred to as a gene repair oligonucleotide) containing a targeted modification to a gene within the genome of the host cell. The polynucleotide modification template for use in such methods can be either single-stranded or double-stranded. Examples of such methods are generally described, for example, in US Publication No. 2013/0019349.

In some embodiments, gene editing may be facilitated through the induction of a double-stranded break (DSB) in a defined position in the genome near the desired alteration. DSBs can be induced using any DSB-inducing agent available, including, but not limited to, TALENs, meganucleases, zinc finger nucleases, Cas9-gRNA systems (based on bacterial CRISPR-Cas systems), and the like. In some embodiments, the introduction of a DSB can be combined with the introduction of a polynucleotide modification template.

The process for editing a genomic sequence combining DSB and modification templates generally comprises: providing to a host cell, a DSB-inducing agent, or a nucleic acid encoding a DSB-inducing agent, that recognizes a target sequence in the chromosomal sequence and is able to induce a DSB in the genomic sequence, and at least one polynucleotide modification template comprising at least one nucleotide alteration when compared to the nucleotide sequence to be edited. The polynucleotide modification template can further comprise nucleotide sequences flanking the at least one nucleotide alteration, in which the flanking sequences are substantially homologous to the chromosomal region flanking the DSB. Genome editing using DSB-inducing agents, such as Cas9-gRNA complexes, has been described, for example in U.S. Patent Application US 2015/0082478, published on March 19, 2015 and WO 2015/026886, published on February 26, 2015.

The terms "knock-in", "gene knock-in", "gene insertion" and "genetic knock-in" are used interchangeably herein. A knock-in represents the replacement or insertion of a DNA sequence at a specific DNA sequence in cell by targeting with a Cas protein (by HR, wherein a suitable donor DNA polynucleotide is also used). Examples of knock-ins are a specific insertion of a heterologous amino acid coding sequence in a coding region of a gene, or a specific insertion of a transcriptional regulatory element in a genetic locus.

Various methods and compositions can be employed to obtain a cell or organism having a polynucleotide of interest inserted in a target site for a Cas endonuclease. Such methods can employ homologous recombination to provide integration of the polynucleotide of Interest at the target site. In one method provided, a polynucleotide of interest is provided to the organism cell in a donor DNA construct. As used herein, "donor DNA" is a DNA construct that comprises a polynucleotide of Interest to be inserted into the target site of a Cas endonuclease. The donor DNA construct further comprises a first and a second region of homology that flank the polynucleotide of Interest. The first and second regions of homology of the donor DNA share homology to a first and a second genomic region, respectively, present in or flanking the target site of the cell or organism genome. By "homology" is meant DNA sequences that are similar. For example, a "region of homology to a genomic region" that is found on the donor DNA is a region of DNA that has a similar sequence to a given "genomic region" in the cell or organism genome. A region of homology can be of any length that is sufficient to promote homologous recombination at the cleaved target site. For example, the region of homology can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800, 5-2900, 5-3000, 5-3100 or more bases in length such that the region of homology has sufficient homology to undergo homologous recombination with the corresponding genomic region. "Sufficient homology" indicates that two polynucleotide sequences have sufficient structural similarity to act as substrates for a homologous recombination reaction. The structural similarity includes overall length of each polynucleotide fragment, as well as the sequence similarity of the polynucleotides. Sequence similarity can be described by the percent sequence identity over the whole length of the sequences, and/or by conserved regions comprising localized similarities such as contiguous nucleotides having 100% sequence identity, and percent sequence identity over a portion of the length of the sequences.

The amount of homology or sequence identity shared by a target and a donor polynucleotide can vary and includes total lengths and/or regions having unit integral values in the ranges of about 1-20 bp, 20-50 bp, 50-100 bp, 75-150 bp, 100-250 bp, 150-300 bp, 200-400 bp, 250-500 bp, 300-600 bp, 350-750 bp, 400-800 bp, 450-900 bp, 500-1000 bp, 600-1250 bp, 700-1500 bp, 800-1750 bp, 900-2000 bp, 1-2.5 kb, 1.5-3 kb, 2-4 kb, 2.5-5 kb, 3-6 kb, 3.5-7 kb, 4-8 kb, 5-10 kb, or up to and including the total length of the target site. These ranges include every integer within the range, for example, the range of 1-20 bp includes 1, 2, 3, 4, 5, 6, 7, 8, 9, 10, 11, 12, 13, 14, 15, 16, 17, 18, 19 and 20 bps. The amount of homology can also described by percent sequence identity over the full aligned length of the two polynucleotides which includes percent sequence identity of about at least 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Sufficient homology includes any combination of polynucleotide length, global percent sequence identity, and optionally conserved regions of contiguous nucleotides or local percent sequence identity, for example sufficient homology can be described as a region of 75-150 bp having at least 80% sequence identity to a region of the target locus. Sufficient homology can also be described by the predicted ability of two polynucleotides to specifically hybridize under high stringency conditions, see, for example, Sambrook et al., (1989) Molecular Cloning: A Laboratory Manual, (Cold Spring Harbor Laboratory Press, NY); Current Protocols in Molecular Biology, Ausubel et al., Eds (1994) Current Protocols, (Greene Publishing Associates, Inc. and John Wiley & Sons, Inc.); and, Tijssen (1993) Laboratory Techniques in Biochemistry and Molecular Biology--Hybridization with Nucleic Acid Probes, (Elsevier, New York).

As used herein, a "genomic region" is a segment of a chromosome in the genome of a cell that is present on either side of the target site or, alternatively, also comprises a portion of the target site. The genomic region can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800. 5-2900, 5-3000, 5-3100 or more bases such that the genomic region has sufficient homology to undergo homologous recombination with the corresponding region of homology.

Polynucleotides of interest and/or traits can be stacked together in a complex trait locus as described in US 2013/0263324, published October 3, 2013 and in WO 2013/112686, published January 24, 2013. The guide polynucleotide/Cas9 endonuclease system described herein provides for an efficient system to generate double strand breaks and allows for traits to be stacked in a complex trait locus.

The structural similarity between a given genomic region and the corresponding region of homology found on the donor DNA can be any degree of sequence identity that allows for homologous recombination to occur. For example, the amount of homology or sequence identity shared by the "region of homology" of the donor DNA and the "genomic region" of the organism genome can be at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity, such that the sequences undergo homologous recombination

The region of homology on the donor DNA can have homology to any sequence flanking the target site. While in some embodiments the regions of homology share significant sequence homology to the genomic sequence immediately flanking the target site, it is recognized that the regions of homology can be designed to have sufficient homology to regions that may be further 5' or 3' to the target site. In still other embodiments, the regions of homology can also have homology with a fragment of the target site along with downstream genomic regions. In one embodiment, the first region of homology further comprises a first fragment of the target site and the second region of homology comprises a second fragment of the target site, wherein the first and second fragments are dissimilar.

As used herein, "homologous recombination" includes the exchange of DNA fragments between two DNA molecules at the sites of homology. The frequency of homologous recombination is influenced by a number of factors. Different organisms vary with respect to the amount of homologous recombination and the relative proportion of homologous to non-homologous recombination. Generally, the length of the region of homology affects the frequency of homologous recombination events: the longer the region of homology, the greater the frequency. The length of the homology region needed to observe homologous recombination is also speciesvariable. In many cases, at least 5 kb of homology has been utilized, but homologous recombination has been observed with as little as 25-50 bp of homology. See, for example, Singer et al., (1982) Cell 31:25-33; Shen and Huang, (1986) Genetics 112:441-57; Watt et al., (1985) Proc. Natl. Acad. Sci. USA 82:4768-72, Sugawara and Haber, (1992) Mol Cell Biol 12:563-75, Rubnitz and Subramani, (1984) Mol Cell Biol 4:2253-8; Ayares et al., (1986) Proc. Natl. Acad. Sci. USA 83:5199-203; Liskay et al., (1987) Genetics 115:161-7.

Homology-directed repair (HDR) is a mechanism in cells to repair double-stranded and single stranded DNA breaks. Homology-directed repair includes homologous recombination (HR) and single-strand annealing (SSA) (Lieber. 2010 Annu. Rev. Biochem. 79:181-211). The most common form of HDR is called homologous recombination (HR), which has the longest sequence homology requirements between the donor and acceptor DNA. Other forms of HDR include single-stranded annealing (SSA) and breakage-induced replication, and these require shorter sequence homology relative to HR. Homology-directed repair at nicks (single-stranded breaks) can occur via a mechanism distinct from HDR at double-strand breaks (Davis and Maizels. (2014) PNAS (0027-8424), 111 (10), p. E924-E932).

Alteration of the genome of a plant cell, for example, through homologous recombination (HR), is a powerful tool for genetic engineering. Homologous recombination has been demonstrated in plants (Halfter et al., (1992) Mol Gen Genet 231:186-93) and insects (Dray and Gloor, 1997, Genetics 147:689-99). Homologous recombination has also been accomplished in other organisms. For example, at least 150-200 bp of homology was required for homologous recombination in the parasitic protozoan Leishmania (Papadopoulou and Dumas, (1997) Nucleic Acids Res 25:4278-86). In the filamentous fungus *Aspergillus nidulans,* gene replacement has been accomplished with as little as 50 bp flanking homology (Chaveroche et al., (2000) Nucleic Acids Res 28:e97). Targeted gene replacement has also been demonstrated in the ciliate *Tetrahymena thermophila* (Gaertig et al., (1994) Nucleic Acids Res 22:5391-8). In mammals, homologous recombination has been most successful in the mouse using pluripotent embryonic stem cell lines (ES) that can be grown in culture, transformed, selected and introduced into a mouse embryo (Watson et al., 1992, Recombinant DNA, 2nd Ed., (Scientific American Books distributed by WH Freeman & Co.).

Error-prone DNA repair mechanisms can produce mutations at double-strand break sites. The Non-Homologous-End-Joining (NHEJ) pathways are the most common repair mechanism to bring the broken ends together (Bleuyard et al., (2006) DNA Repair 5:1-12). The structural integrity of chromosomes is typically preserved by the repair, but deletions, insertions, or other rearrangements are possible. The two ends of one double-strand break are the most prevalent substrates of NHEJ (Kirik et al., (2000) EMBO J 19:5562-6), however if two different double-strand breaks occur, the free ends from different breaks can be ligated and result in chromosomal deletions (Siebert and Puchta, (2002) Plant Cell 14:1121-31), or chromosomal translocations between different chromosomes (Pacher et al., (2007) Genetics 175:21-9). Microhomology-mediated end joining MMEH is described in US patent application US 2014/0242702, published on August 28, 2014.

It is understood by anyone skilled in the art that the Cas endonuclease used in the methods described herein can be substituted by any double strand break inducing agent such us but not limited to TAL nucleases (TALENs), designer zinc-finger nucleases, engineered meganucleases and homing meganucleases.

Episomal DNA molecules can also be ligated into the double-strand break, for example, integration of T-DNAs into chromosomal double-strand breaks (Chilton and Que, (2003) Plant Physiol 133:956-65; Salomon and Puchta, (1998) EMBO J 17:6086-95). Once the sequence around the double-strand breaks is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

Once a double-strand break is induced in the DNA, the cell's DNA repair mechanism is activated to repair the break. Error-prone DNA repair mechanisms can produce mutations at double-strand break sites. The most common repair mechanism to bring the broken ends together is the nonhomologous end-joining (NHEJ) pathway (Bleuyard et al., (2006) DNA Repair 5:1-12). The structural integrity of chromosomes is typically preserved by the repair, but deletions, insertions, or other rearrangements are possible (Siebert and Puchta, (2002) Plant Cell 14:1121-31; Pacher et al., (2007) Genetics 175:21-9).

Alternatively, the double-strand break can be repaired by homologous recombination between homologous DNA sequences. Once the sequence around the double-strand break is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

DNA double-strand breaks appear to be an effective factor to stimulate homologous recombination pathways (Puchta et al., (1995) Plant Mol Biol 28:281-92; Tzfira and White, (2005) Trends Biotechnol 23:567-9; Puchta, (2005) J Exp Bot 56:1-14). Using DNA-breaking agents, a two- to nine-fold increase of homologous recombination was observed between artificially constructed homologous DNA repeats in plants (Puchta et al., (1995) Plant Mol Biol 28:281-92). In maize protoplasts, experiments with linear DNA molecules demonstrated enhanced homologous recombination between plasmids (Lyznik et al., (1991) Mol Gen Genet 230:209-18).

The donor DNA may be introduced by any means known in the art. The donor DNA may be provided by any transformation method known in the art including, for example, Agrobacterium-mediated transformation, biolistic particle bombardment, chemical transformation, protoplast fusion, or electroporation. The donor DNA may be present transiently in the cell or it could be introduced via a bacterial, yeast, fungal, or viral replicon. In the presence of the Cas endonuclease and the target site, the donor DNA is inserted into the transformed cell's genome.

Further uses for guide RNA/Cas endonuclease systems have been described (see U.S. Patent Application US 2015/0082478, published on March 19, 2015, WO 2015/026886, published on February 26, 2015 and US 2015/0059010, published on February 26, 2015) and include but are not limited to modifying or replacing nucleotide sequences of interest (such as a regulatory elements), insertion of polynucleotides of interest, gene knock-out, gene knock-in, modification of splicing sites and/or introducing alternate splicing sites, modifications of nucleotide sequences encoding a protein of interest, amino acid and/or protein fusions, and gene silencing by expressing an inverted repeat into a gene of interest.

Polynucleotides of interest are further described herein and include polynucleotides reflective of the commercial markets and interests of those involved in the development of the crop. Polynucleotides/polypeptides of interest include, but are not limited to, herbicide-resistance coding sequences, insecticidal coding sequences, nematicidal coding sequences, antimicrobial coding sequences, antifungal coding sequences, antiviral coding sequences, abiotic and biotic stress tolerance coding sequences, or sequences modifying microbial or plant traits such as yield, grain quality, nutrient content, starch quality and quantity, nitrogen fixation and/or utilization, fatty acids, and oil content and/or composition.

Furthermore, it is recognized that the polynucleotide of interest may also comprise antisense sequences complementary to at least a portion of the messenger RNA (mRNA) for a targeted gene sequence of interest. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, 80%, or 85% sequence identity to the corresponding antisense sequences may be used. Furthermore, portions of the antisense nucleotides may be used to disrupt the expression of the target gene. Generally, sequences of at least 50 nucleotides, 100 nucleotides, 200 nucleotides, or greater may be used.

In addition, the polynucleotide of interest may also be used in the sense orientation to suppress the expression of endogenous genes in plants and microbes. Methods for suppressing gene expression in plants and microbes using polynucleotides in the sense orientation are known in the art. The methods generally involve transforming plants or microbes with a DNA construct comprising a promoter that drives expression in a microbe or plant operably linked to at least a portion of a nucleotide sequence that corresponds to the transcript of the endogenous gene. Typically, such a nucleotide sequence has substantial sequence identity to the sequence of the transcript of the endogenous gene, generally greater than about 65% sequence identity, about 85% sequence identity, or greater than about 95% sequence identity. See, U.S. Patent Nos. 5,283,184 and 5,034,323.

The polynucleotide of interest can also be a phenotypic marker. A phenotypic marker is screenable or a selectable marker that includes visual markers and selectable markers whether it is a positive or negative selectable marker. Any phenotypic marker can be used. Specifically, a selectable or screenable marker comprises a DNA segment that allows one to identify, or select for or against a molecule or a cell that contains it, often under particular conditions. These markers can encode an activity, such as, but not limited to, production of RNA, peptide, or protein, or can provide a binding site for RNA, peptides, proteins, inorganic and organic compounds or compositions and the like.

As used herein, "nucleic acid" means a polynucleotide and includes a single or a double-stranded polymer of deoxyribonucleotide or ribonucleotide bases. Nucleic acids may also include fragments and modified nucleotides. Thus, the terms "polynucleotide", "nucleic acid sequence", "nucleotide sequence" and "nucleic acid fragment" are used interchangeably to denote a polymer of RNA and/or DNA that is single- or double-stranded, optionally containing synthetic, non-natural, or altered nucleotide bases. Nucleotides (usually found in their 5'-monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide.

"Open reading frame" is abbreviated ORF.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of genes to produce the desired phenotype in a transformed plant. Genes can be designed for use in suppression by linking a nucleic acid fragment or subfragment thereof, whether or not it encodes an active enzyme, in the sense or antisense orientation relative to a plant promoter sequence.

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential to the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

Polynucleotide and polypeptide sequences, variants thereof, and the structural relationships of these sequences can be described by the terms "homology", "homologous", "substantially identical", "substantially similar" and "corresponding substantially" which are used interchangeably herein. These refer to polypeptide or nucleic acid fragments wherein changes in one or more amino acids or nucleotide bases do not affect the function of the molecule, such as the ability to mediate gene expression or to produce a certain phenotype. These terms also refer to modification(s) of nucleic acid fragments that do not substantially alter the functional properties of the resulting nucleic acid fragment relative to the initial, unmodified fragment. These modifications include deletion, substitution, and/or insertion of one or more nucleotides in the nucleic acid fragment.

Substantially similar nucleic acid sequences encompassed may be defined by their ability to hybridize (under moderately stringent conditions, e.g., 0.5X SSC, 0.1% SDS, 60°C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, or 90% sequence identity, up to and including 100% sequence identity (i.e., fully complementary) with each other.

The term "stringent conditions" or "stringent hybridization conditions" includes reference to conditions under which a probe will selectively hybridize to its target sequence in an *in vitro* hybridization assay. Stringent conditions are sequencedependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salt(s)) at pH 7.0 to 8.3, and at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C.

"Sequence identity" or "identity" in the context of nucleic acid or polypeptide sequences refers to the nucleic acid bases or amino acid residues in two sequences that are the same when aligned for maximum correspondence over a specified comparison window.

The term "percentage of sequence identity" refers to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90% or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign^{™} program of the LASERGENE bioinformatics computing suite (DNASTAR Inc., Madison, WI). Within the context of this patent it will be understood that where sequence analysis software is used for analysis, that the results of the analysis will be based on the "default values" of the program referenced, unless otherwise specified. As used herein "default values" will mean any set of values or parameters that originally load with the software when first initialized.

The "Clustal V method of alignment" corresponds to the alignment method labeled Clustal V (described by Higgins and Sharp, (1989) CABIOS 5:151-153; Higgins et al., (1992) Comput Appl Biosci 8:189-191) and found in the MegAlign^{™} program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

The "Clustal W method of alignment" corresponds to the alignment method labeled Clustal W (described by Higgins and Sharp, (1989) CABIOS 5:151-153; Higgins et al., (1992) Comput Appl Biosci 8:189-191) and found in the MegAlign^{™} v6.1 program of the LASERGENE bioinformatics computing suite (DNASTAR Inc., Madison, WI). Default parameters for multiple alignment (GAP PENALTY=10, GAP LENGTH PENALTY=0.2, Delay Divergen Seqs (%)=30, DNA Transition Weight=0.5, Protein Weight Matrix=Gonnet Series, DNA Weight Matrix=IUB ). After alignment of the sequences using the Clustal W program, it is possible to obtain a "percent identity" by viewing the "sequence distances" table in the same program.

Unless otherwise stated, sequence identity/similarity values provided herein refer to the value obtained using GAP Version 10 (GCG, Accelrys, San Diego, CA) using the following parameters: % identity and % similarity for a nucleotide sequence using a gap creation penalty weight of 50 and a gap length extension penalty weight of 3, and the nwsgapdna.cmp scoring matrix; % identity and % similarity for an amino acid sequence using a GAP creation penalty weight of 8 and a gap length extension penalty of 2, and the BLOSUM62 scoring matrix (Henikoff and Henikoff, (1989) Proc. Natl. Acad. Sci. USA 89:10915). GAP uses the algorithm of Needleman and Wunsch,

(1970) J Mol Biol 48:443-53, to find an alignment of two complete sequences that maximizes the number of matches and minimizes the number of gaps. GAP considers all possible alignments and gap positions and creates the alignment with the largest number of matched bases and the fewest gaps, using a gap creation penalty and a gap extension penalty in units of matched bases.

"BLAST" is a searching algorithm provided by the National Center for Biotechnology Information (NCBI) used to find regions of similarity between biological sequences. The program compares nucleotide or protein sequences to sequence databases and calculates the statistical significance of matches to identify sequences having sufficient similarity to a query sequence such that the similarity would not be predicted to have occurred randomly. BLAST reports the identified sequences and their local alignment to the query sequence.

It is well understood by one skilled in the art that many levels of sequence identity are useful in identifying polypeptides from other species or modified naturally or synthetically wherein such polypeptides have the same or similar function or activity. Useful examples of percent identities include, but are not limited to, 50%, 55%, 60%, 65%, 70%, 75%, 80%, 85%, 90% or 95%, or any integer percentage from 50% to 100%. Indeed, any integer amino acid identity from 50% to 100% may be useful in describing the present disclosure, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

"Gene" includes a nucleic acid fragment that expresses a functional molecule such as, but not limited to, a specific protein, including regulatory sequences preceding (5' non-coding sequences) and following (3' non-coding sequences) the coding sequence. "Native gene" refers to a gene as found in nature with its own regulatory sequences.

A "mutated gene" is a gene that has been altered through human intervention. Such a "mutated gene" has a sequence that differs from the sequence of the corresponding non-mutated gene by at least one nucleotide addition, deletion, or substitution. In certain embodiments of the disclosure, the mutated gene comprises an alteration that results from a guide polynucleotide/Cas endonuclease system as disclosed herein. A mutated plant is a plant comprising a mutated gene.

As used herein, a "targeted mutation" is a mutation in a native gene that was made by altering a target sequence within the native gene using a method involving a double-strand-break-inducing agent that is capable of inducing a double-strand break in the DNA of the target sequence as disclosed herein or known in the art.

The guide RNA/Cas endonuclease induced targeted mutation can occur in a nucleotide sequence that is located within or outside a genomic target site that is recognized and cleaved by a Cas endonuclease.

Mutation efficiency can be calculated as described herein (see Examples). The mutation efficiency caused by a guideRNA /Cas endonuclease system wherein the guide RNA originates from a recombinant DNA expression cassette comprising a Pol-II promoter operably linked to a polynucleotide encoding a single guide RNA, wherein said guide RNA is capable of forming a guide RNA/Cas endonuclease complex, wherein said complex can bind to and cleave a target site sequence in the genome of a non-conventional yeast, can be compared to mutation frequencies casused by a guideRNA /Cas endonuclease system wherein the guide RNA originates from recombinant DNA constructs not comprising a Poll pormoter.

The term "genome" as it applies to a plant, yeast of fungalcell encompasses not only chromosomal DNA found within the nucleus, but organelle DNA found within subcellular components (e.g., mitochondria, or plastid) of the cell.

A "codon-modified gene" or "codon-preferred gene" or "codon-optimized gene" is a gene having its frequency of codon usage designed to mimic the frequency of preferred codon usage of the host cell.

An "allele" is one of several alternative forms of a gene occupying a given locus on a chromosome. When all the alleles present at a given locus on a chromosome are the same, that cell is homozygous at that locus. If the alleles present at a given locus on a chromosome differ, that cell is heterozygous at that locus.

"Coding sequence" refers to a polynucleotide sequence which codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to: promoters, translation leader sequences, 5' untranslated sequences, 3' untranslated sequences, introns, polyadenylation target sequences, RNA processing sites, effector binding sites, and stem-loop structures.

"A plant-optimized nucleotide sequence" is nucleotide sequence that has been optimized for increased expression in plants, particularly for increased expression in plants or in one or more plants of interest. For example, a plant-optimized nucleotide sequence can be synthesized by modifying a nucleotide sequence encoding a protein such as, for example, double-strand-break-inducing agent (e.g., an endonuclease) as disclosed herein, using one or more plant-preferred codons for improved expression. See, for example, Campbell and Gowri (1990) Plant Physiol. 92:1-11 for a discussion of host-preferred codon usage.

Methods are available in the art for synthesizing plant-preferred genes. See, for example, U.S. Patent Nos. 5,380,831, and 5,436,391, and Murray et al. (1989) Nucleic Acids Res. 17:477-498. Additional sequence modifications are known to enhance gene expression in a plant host. These include, for example, elimination of: one or more sequences encoding spurious polyadenylation signals, one or more exonintron splice site signals, one or more transposon-like repeats, and other such wellcharacterized sequences that may be deleterious to gene expression. The G-C content of the sequence may be adjusted to levels average for a given plant host, as calculated by reference to known genes expressed in the host plant cell. When possible, the sequence is modified to avoid one or more predicted hairpin secondary mRNA structures. Thus, "a plant-optimized nucleotide sequence" of the present disclosure comprises one or more of such sequence modifications.

A promoter is a region of DNA involved in recognition and binding of RNA polymerase and other proteins to initiate transcription. The promoter sequence consists of proximal and more distal upstream elements, the latter elements often referred to as enhancers. An "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, and/or comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters". It has been shown that certain promoters are able to direct RNA synthesis at a higher rate than others. These are called "strong promoters". Certain other promoters have been shown to direct RNA synthesis at higher levels only in particular types of cells or tissues and are often referred to as "tissue specific promoters", or "tissue-preferred promoters" if the promoter directs RNA synthesis preferably in certain tissues but also in other tissues at reduced levels. Since patterns of expression of a chimeric gene (or genes) introduced into a plant are controlled using promoters, there is an ongoing interest in the isolation of novel promoters which are capable of controlling the expression of a chimeric gene or (genes) at certain levels in specific tissue types or at specific plant developmental stages.

Chemical-regulated promoters can be used to modulate the expression of a gene in a plant through the application of an exogenous chemical regulator. The promoter may be a chemical-inducible promoter, where application of the chemical induces gene expression, or a chemical-repressible promoter, where application of the chemical represses gene expression (De Veylder et al., (1997) Plant Cell Physiol 38:568-77). Tissue-preferred promoters can be utilized to target enhanced expression within a particular plant tissue (Kawamata et al., (1997) Plant Cell Physiol38:792-803). Seed-preferred promoters include both seed-specific promoters active during seed development, as well as seed-germinating promoters active during seed germination (Thompson et al., 1989, BioEssays 10:108).

The term "inducible promoter" refers to promoters that selectively express a coding sequence or functional RNA in response to the presence of an endogenous or exogenous stimulus, for example by chemical compounds (chemical inducers) or in response to environmental, hormonal, chemical, and/or developmental signals. Inducible or regulated promoters include, for example, promoters induced or regulated by light, heat, stress, flooding or drought, salt stress, osmotic stress, phytohormones, wounding, or chemicals such as ethanol, abscisic acid (ABA), jasmonate, salicylic acid, or safeners.

Examples of strong promoters useful in certain aspects herein (e.g., fungal and/or yeast cells) include those disclosed in U.S. Patent Appl. Publ. Nos. 2012/0252079 (DGAT2), 2012/0252093 (EL1), 2013/0089910 (ALK2), 2013/0089911 (SPS19), 2006/0019297 (GPD and GPM), 2011/0059496 (GPD and GPM), 2005/0130280 (FBA, FBAIN, FBAINm), 2006/0057690 (GPAT) and 2010/0068789 (YAT1). Other examples of strong promoters include XPR2 (U.S. Pat. No. 4937189; EP 220864), GPD, GPM (U.S. Pat. Nos. 7259255 and 7459546),TEF (U.S. Pat. No. 6265185), GPDIN (U.S. Pat. No. 7459546, GPM/FBAIN (U.S. Pat. No. 7202356), FBA, FBAIN, FBAINm (U.S. Pat. No. 7202356), GPAT (U.S. Pat. No. 7264949), YAT1 (U.S. Pat. Appl. Publ. No. 2006/0094102) and EXP1 (U.S. Pat. No. 7932077). Other examples of strong promoters useful in certain embodiments herein include PGK1, ADH1, TDH3, TEF1, PHO5, LEU2, and GAL1 promoters, as well as strong yeast promoters disclosed in Velculescu et al. (Cell 88:243-251).

A tRNA promoter includes a DNA encoding any one tRNA known in the art such as but limiting to tRNA-Lysine (tRNA-Lys; see Acker et al. 2008. Nucleic acid res. 36(18):5832-5844), a tRNA-Glutamine (tRNA-Glu), a tRNA-Valine (tRNA Val; Marck et al. 2006. Nuceic Acid Res. 34(6):1816-1835) or any other tRNA active in a cell, a tRNA-leucine (tRNA Leu, tRNA-leu(2), tRNA-leu(3)), a tRNA-isoleucine (tRNA-ile), a tRNA-tryptophan (tRNA-trp), a tRNA-tyrosine (tRNA-tyr), a tRNA-histidine (tRNA-his; tRNA-his).

"Translation leader sequence" refers to a polynucleotide sequence located between the promoter sequence of a gene and the coding sequence. The translation leader sequence is present in the mRNA upstream of the translation start sequence. The translation leader sequence may affect processing of the primary transcript to mRNA, mRNA stability or translation efficiency. Also referred to as 5' untranslated region. Examples of translation leader sequences have been described (e.g., Turner and Foster, (1995) Mol Biotechnol 3:225-236).

"3' non-coding sequences", "transcription terminator" or "termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) Plant Cell 1:671-680.

"RNA transcript" refers to the product resulting from RNA polymerase-catalyzed transcription of a DNA sequence. When the RNA transcript is a perfect complimentary copy of the DNA sequence, it is referred to as the primary transcript or pre-mRNA. A RNA transcript is referred to as the mature RNA or mRNA when it is a RNA sequence derived from post-transcriptional processing of the primary transcript pre mRNAt. "Messenger RNA" or "mRNA" refers to the RNA that is without introns and that can be translated into protein by the cell. "cDNA" refers to a DNA that is complementary to, and synthesized from, a mRNA template using the enzyme reverse transcriptase. The cDNA can be single-stranded or converted into double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that blocks the expression of a target gene (see, e.g., U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., the coding sequence is under the transcriptional control of the promoter). Coding sequences can be operably linked to regulatory sequences in a sense or antisense orientation. In another example, the complementary RNA regions can be operably linked, either directly or indirectly, 5' to the target mRNA, or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook et al., Molecular Cloning: A Laboratory Manual; Cold Spring Harbor Laboratory: Cold Spring Harbor, NY (1989). Transformation methods are well known to those skilled in the art and are described *infra.*

"PCR" or "polymerase chain reaction" is a technique for the synthesis of specific DNA segments and consists of a series of repetitive denaturation, annealing, and extension cycles. Typically, a double-stranded DNA is heat denatured, and two primers complementary to the 3' boundaries of the target segment are annealed to the DNA at low temperature, and then extended at an intermediate temperature. One set of these three consecutive steps is referred to as a "cycle".

The term "recombinant" refers to an artificial combination of two otherwise separated segments of sequence, e.g., by chemical synthesis, or manipulation of isolated segments of nucleic acids by genetic engineering techniques.

The terms "plasmid", "vector" and "cassette" refer to an extra chromosomal element often carrying genes that are not part of the central metabolism of the cell, and usually in the form of double-stranded DNA. Such elements may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. "Transformation cassette" refers to a specific vector containing a gene and having elements in addition to the gene that facilitates transformation of a particular host cell. "Expression cassette" refers to a specific vector containing a gene and having elements in addition to the gene that allow for expression of that gene in a host.

The terms "recombinant DNA molecule", "recombinant construct", "expression construct", "construct", "construct", and "recombinant DNA construct" are used interchangeably herein. A recombinant construct comprises an artificial combination of nucleic acid fragments, e.g., regulatory and coding sequences that are not all found together in nature. For example, a construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et al., (1985) EMBO J 4:2411-2418; De Almeida et al., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The term "expression", as used herein, refers to the production of a functional end-product (e.g., an mRNA, guide RNA, or a protein) in either precursor or mature form.

The term "providing" includes providing a nucleic acid (e.g., expression construct) or peptide, polypeitde or protein to a cell. Providing includes reference to the incorporation of a nucleic acid or polypeptide into a eukaryotic or prokaryotic cell where the nucleic acid may be incorporated into the genome of the cell, and includes reference to the transient provision of a nucleic acid or protein to the cell. Providing includes reference to stable or transient transformation methods, transfection, transduction, microinjection, electroporation, viral methods, *Agrobacterium-mediated* transformation, ballistic particle acceleration as well as sexually crossing. Thus, "providing" in the context of inserting a nucleic acid fragment (e.g., a recombinant DNA construct/expression construct, guide RNA, guide DNA, template DNA, donor DNA) into a cell, includes "transfection" or "transformation" or "transduction" and includes reference to the incorporation of a nucleic acid fragment into a eukaryotic or prokaryotic cell where the nucleic acid fragment may be incorporated into the genome of the cell (e.g., chromosome, plasmid, plastid, or mitochondrial DNA), converted into an autonomous replicon, or transiently expressed (e.g., transfected mRNA).

A variety of methods are known for contacting, providing, and/or introducing a composition (such as a nucleotide sequence, a peptide or a polypeptide) into an organism including stable transformation methods, transient transformation methods, virus-mediated methods, sexual crossing and sexual breeding. Stable transformation indicates that the introduced polynucleotide integrates into the genome of the organism and is capable of being inherited by progeny thereof. Transient transformation indicates that the introduced composition is only temporarily expressed or present in the organism.

Protocols for contacting, providing, introducing polynucleotides and polypeptides to cells or organisms are known and include microinjection (Crossway et al., (1986) Biotechniques 4:320-34 and U.S. Patent No. 6,300,543), meristem transformation (U.S. Patent No. 5,736,369), electroporation (Riggs et al., (1986) Proc. Natl. Acad. Sci. USA 83:5602-6, *Agrobacterium-mediated* transformation (U.S. Patent Nos. 5,563,055 and 5,981,840), direct gene transfer (Paszkowski et al., (1984) EMBO J 3:2717-22), and ballistic particle acceleration (U.S. Patent Nos. 4,945,050; 5,879,918; 5,886,244; 5,932,782; Tomes et al., (1995) "Direct DNA Transfer into Intact Plant Cells via Microprojectile Bombardment" in Plant Cell, Tissue, and Organ Culture: Fundamental Methods, ed. Gamborg & Phillips (Springer-Verlag, Berlin); McCabe et al., (1988) Biotechnology 6:923-6; Weissinger et al., (1988) Ann Rev Genet 22:421-77; Sanford et al., (1987) Particulate Science and Technology 5:27-37 (onion); Christou et al., (1988) Plant Physiol 87:671-4 (soybean); Finer and McMullen, (1991) In Vitro Cell Dev Biol 27P:175-82 (soybean); Singh et al., (1998) Theor Appl Genet 96:319-24 (soybean); Datta et al., (1990) Biotechnology 8:736-40 (rice); Klein et al., (1988) Proc. Natl. Acad. Sci. USA 85:4305-9 (maize); Klein et al., (1988) Biotechnology 6:559-63 (maize); U.S. Patent Nos. 5,240,855; 5,322,783 and 5,324,646; Klein et al., (1988) Plant Physiol 91:440-4 (maize); Fromm et al., (1990) Biotechnology 8:833-9 (maize); Hooykaas-Van Slogteren et al., (1984) Nature 311:763-4; U.S. Patent No. 5,736,369 (cereals); Bytebier et al., (1987) Proc. Natl. Acad. Sci. USA 84:5345-9 (*Liliaceae*); De Wet et al., (1985) in The Experimental Manipulation of Ovule Tissues, ed. Chapman et al., (Longman, New York), pp. 197-209 (pollen); Kaeppler et al., (1990) Plant Cell Rep 9:415-8) and Kaeppler et al., (1992) Theor Appl Genet 84:560-6 (whisker-mediated transformation); D'Halluin et al., (1992) Plant Cell 4:1495-505 (electroporation); Li et al., (1993) Plant Cell Rep 12:250-5; Christou and Ford (1995) Annals Botany 75:407-13 (rice) and Osjoda et al., (1996) Nat Biotechnol 14:745-50 (maize via *Agrobacterium tumefaciens*).

Alternatively, polynucleotides may be introduced into cells or organisms by contacting cells or organisms with a virus or viral nucleic acids. Generally, such methods involve incorporating a polynucleotide within a viral DNA or RNA molecule. In some examples a polypeptide of interest may be initially synthesized as part of a viral polyprotein, which is later processed by proteolysis *in vivo* or *in vitro* to produce the desired recombinant protein. Methods for introducing polynucleotides into plants and expressing a protein encoded therein, involving viral DNA or RNA molecules, are known, see, for example, U.S. Patent Nos. 5,889,191, 5,889,190, 5,866,785, 5,589,367 and 5,316,931. Transient transformation methods include, but are not limited to, the introduction of polypeptides, such as a double-strand break inducing agent, directly into the organism, the introduction of polynucleotides such as DNA and/or RNA polynucleotides, and the introduction of the RNA transcript, such as an mRNA encoding a double-strand break inducing agent, into the organism. Such methods include, for example, microinjection or particle bombardment. See for example Crossway et al., (1986) Mol Gen Genet 202:179-85; Nomura et al., (1986) Plant Sci 44:53-8; Hepler et al., (1994) Proc. Natl. Acad. Sci. USA 91:2176-80; and, Hush et al., (1994) J Cell Sci 107:775-84.

Nucleid acids and proteins can be provided to a cell by any method, including methods using molecules to facilitate the uptake of any one or all components of a guided Cas system (protein and/or nucleic acids), such as cell-penetrating peptides and nanocarriers. See also US 2011/0035836 ("Nanocarrier based plant transfection and transduction") and EP 2821486 ("Method of introducing nucleic acid into plant cells").

Providing a guide RNA/Cas endonuclease complex to a cell includes providing the individual components of said complex to the cell either directly or via recombination constructs, and includes providing the whole complex to the cell as well.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or other DNA-containing organelle, of a host organism resulting in gene expression without integration or stable inheritance. Host organisms containing the transformed nucleic acid fragments are referred to as "transgenic" organisms.

The term "cell" herein refers to any type of cell such as a prokaryotic or eukaryotic cell. A eukaryotic cell has a nucleus and other membrane-enclosed structures (organelles), whereas a prokaryotic cell lacks a nucleus. A cell in certain embodiments can be a mammalian cell or non-mammalian cell. Non-mammalian cells can be eukaryotic or prokaryotic. For example, a non-mammalian cell herein can refer to a microbial cell or cell of a non-mammalian multicellular organism such as a plant, insect, nematode, avian species, amphibian, reptile, or fish.

The terms "control cell" and "suitable control cell" are used interchangeably herein and may be referenced with respect to a cell in which a particular modification (e.g., over-expression of a polynucleotide, down-regulation of a polynucleotide) has been made (i.e., an "experimental cell"). A control cell may be any cell that does not have or does not express the particular modification of the experimental cell. Thus, a control cell may be an untransformed wild type cell or may be genetically transformed but does not express the genetic transformation. For example, a control cell may be a direct parent of the experimental cell, which direct parent cell does not have the particular modification that is in the experimental cell. Alternatively, a control cell may be a parent of the experimental cell that is removed by one or more generations. Alternatively, a control cell may be a sibling of the experimental cell, which sibling does not comprise the particular modification that is present in the experimental cell.

A microbial cell herein can refer to a fungal cell (e.g., yeast cell), prokaryotic cell, protist cell (e.g., algal cell), euglenoid cell, stramenopile cell, or oomycete cell, for example. A prokaryotic cell herein can refer to a bacterial cell or archaeal cell, for example. Fungal cells (e.g., yeast cells), protist cells (e.g., algal cells), euglenoid cells, stramenopile cells, and oomycete cells represent examples of eukaryotic microbial cells. A eukaryotic microbial cell has a nucleus and other membrane-enclosed structures (organelles), whereas a prokaryotic cell lacks a nucleus.

The term "yeast" herein refers to fungal species that predominantly exist in unicellular form. Yeast can alternatively be referred to as "yeast cells". A yeast in certain aspects herein can be one that reproduces asexually (anamorphic) or sexually (teleomorphic). While yeast herein typically exist in unicellular form, certain types of these yeast may optionally be able to form pseudohyphae (strings of connected budding cells). In still further aspects, a yeast may be haploid or diploid, and/or may have the ability to exist in either of these ploidy forms. A yeast herein can be characterized as either a conventional yeast or non-conventional yeast, for example.

The term "conventional yeast" ("model yeast") herein generally refers to Saccharomyces or Schizosaccharomyces yeast species. Conventional yeast include yeast that favor homologous recombination (HR) DNA repair processes over repair processes mediated by non-homologous end-joining (NHEJ). Examples of conventional yeast herein include species of the genera *Saccharomyces* (e.g., *S*. *cerevisiae,* which is also known as budding yeast, baker's yeast, and/or brewer's yeast; *S. bayanus; S. boulardii; S. bulderi; S. cariocanus; S. cariocus; S. chevalieri; S. dairenensis; S. ellipsoideus; S. eubayanus; S. exiguus; S. florentinus; S. kluyveri; S. martiniae; S. monacensis; S. norbensis; S. paradoxus; S. pastorianus; S. spencerorum; S. turicensis; S. unisporus; S. uvarum; S. zonatus*) and *Schizosaccharomyces* (e.g., *S. pombe,* which is also known as fission yeast; *S*. *cryophilus; S. japonicus; S. octosporus).*

The term "non-conventional yeast" herein refers to any yeast that is not a *Saccharomyces* (e.g., *S*. *cerevisiae*) or *Schizosaccharomyces* yeast species. Non-conventional yeast are described in Non-Conventional Yeasts in Genetics, Biochemistry and Biotechnology: Practical Protocols (K. Wolf, K.D. Breunig, G. Barth, Eds., Springer-Verlag, Berlin, Germany, 2003). Non-conventional yeast in certain embodiments may additionally (or alternatively) be yeast that favor non-homologous end-joining (NHEJ) DNA repair processes over repair processes mediated by homologous recombination (HR).

Conventional yeasts such as *S*. *cerevisiae* and *S. pombe* typically exhibit specific integration of donor DNA with short flanking homology arms (30-50 bp) with efficiencies routinely over 70%, whereas non-conventional yeasts such as *Pichia pastoris, Pichia stipitis, Hansenula polymorpha, Yarrowia lipolytica* and *Kluyveromyces lactis* usually show specific integration with similarly structured donor DNA at efficiencies of less than 1% (Chen et al., PLoS ONE 8: e57952). Thus, a preference for HR processes can be gauged, for example, by transforming yeast with a suitable donor DNA and determining the degree to which it is specifically recombined with a genomic site predicted to be targeted by the donor DNA. A preference for NHEJ (or low preference for HR), for example, would be manifest if such an assay yielded a high degree of random integration of the donor DNA in the yeast genome. Assays for determining the rate of specific (HR-mediated) and/or random (NHEJ-mediated) integration of DNA in yeast are known in the art (e.g., Ferreira and Cooper, Genes Dev. 18:2249-2254; Corrigan et al., PLoS ONE 8:e69628; Weaver et al., Proc. Natl. Acad. Sci. U.S.A. 78:6354-6358; Keeney and Boeke, Genetics 136:849-856).

Given their low level of HR activity, non-conventional yeast herein can (i) exhibit a rate of specific targeting by a suitable donor DNA having 30-50 bp flanking homology arms of less than about 1%, 2%, 3%, 4%, 5%, 6%, 7%, or 8%, for example, and/or (ii) exhibit a rate of random integration of the foregoing donor DNA of more than about 65%, 66%, 67%, 68%, 69%, 70%, 71%, 72%, 73%, 74%, or 75%, for example. These rates of (i) specific targeting and/or (ii) random integration of a suitable donor DNA can characterize a non-conventional yeast as it exists before being provided an RGEN as disclosed herein. An aim for providing an RGEN to a non-conventional yeast in certain embodiments is to create site-specific DNA single-strand breaks (SSB) or double-strand breaks (DSB) for biasing the yeast toward HR at the specific site. Thus, providing a suitable RGEN in a non-conventional yeast typically should allow the yeast to exhibit an increased rate of HR with a particular donor DNA. Such an increased rate can be at least about 2-, 3-, 4-, 5-, 6-, 7-, 8-, 9-, or 10-fold higher than the rate of HR in a suitable control (e.g., same non-conventional yeast transformed with the same donor DNA, but lacking a suitable RGEN).

A non-conventional yeast herein can be cultivated following any means known in the art, such as described in Non-Conventional Yeasts in Genetics, Biochemistry and Biotechnology: Practical Protocols (K. Wolf, K.D. Breunig, G. Barth, Eds., Springer-Verlag, Berlin, Germany, 2003), Yeasts in Natural and Artificial Habitats (J.F.T. Spencer, D.M. Spencer, Eds., Springer-Verlag, Berlin, Germany, 1997), and/or Yeast Biotechnology: Diversity and Applications (T. Satyanarayana, G. Kunze, Eds., Springer, 2009).

Non-limiting examples of non-conventional yeast herein include yeasts of the following genera: *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces, Pachysolen,* and *Moniliella. A* suitable example of a *Yarrowia* species is *Y*. *lipolytica.* Suitable examples of *Pichia* species include *P. pastoris, P. methanolica, P. stipitis*, *P. anomala* and *P. angusta.* Suitable examples of *Schwanniomyces* species include *S*. *castellii, S. alluvius, S. hominis, S. occidentalis, S*. capriottii, *S. etchellsii, S. polymorphus, S. pseudopolymorphus, S. vanrijiae* and *S*. *yamadae.* Suitable examples of *Kluyveromyces* species include *K. lactis, K. marxianus, K. fragilis, K. drosophilarum, K. thermotolerans, K. phaseolosporus, K. vanudenii, K. waltii, K. africanus* and *K*. *polysporus.* Suitable examples of *Arxula* species include *A. adeninivorans* and *A*. *terrestre.* Suitable examples of *Trichosporon* species include *T. cutaneum, T. capitatum, T. inkin* and *T. beemeri.* Suitable examples of *Candida* species include *C*. *albicans, C. ascalaphidarum, C. amphixiae, C. antarctica, C. apicola, C. argentea, C. atlantica, C. atmosphaerica, C. blattae, C. bromeliacearum, C. carpophila, C. carvajalis, C. cerambycidarum, C. chauliodes, C. corydali, C. dosseyi, C. dubliniensis, C. ergatensis, C. fructus, C. glabrata, C. fermentati, C. guilliermondii, C. haemulonii, C. insectamens, C. insectorum, C. intermedia, C. jeffresii, C. kefyr, C. keroseneae, C. krusei, C. lusitaniae, C. lyxosophila, C. maltosa, C. marina, C. membranifaciens, C. milleri, C. mogii, C. oleophila*, *C. oregonensis, C. parapsilosis, C. quercitrusa, C. rugosa, C. sake, C. shehatea, C. temnochilae, C. tenuis, C. theae, C. tolerans, C. tropicalis, C. tsuchiyae, C. sinolaborantium, C. sojae, C. subhashii, C. viswanathii, C. utilis, C. ubatubensis* and *C. zemplinina.* Suitable examples of *Ustilago* species include *U. avenae, U. esculenta, U. hordei, U. maydis, U. nuda* and *U. tritici.* Suitable examples of *Torulopsis* species include *T. geochares, T. azyma, T. glabrata* and *T. candida.* Suitable examples of *Zygosaccharomyces* species include *Z. bailii, Z. bisporus, Z. cidri, Z. fermentati, Z. florentinus, Z. kombuchaensis, Z. lentus, Z. mellis, Z. microellipsoides, Z. mrakii, Z. pseudorouxii* and *Z*. *rouxii.* Suitable examples of *Trigonopsis* species include *T. variabilis.* Suitable examples of *Cryptococcus* species include *C. laurentii, C. albidus, C. neoformans, C. gattii, C. uniguttulatus, C. adeliensis, C. aerius, C. albidosimilis, C. antarcticus, C. aquaticus, C. ater, C. bhutanensis, C. consortionis, C. curvatus, C. phenolicus, C. skinneri, C. terreus* and *C*. *vishniacci.* Suitable examples of *Rhodotorula* species include *R. acheniorum, R. tula, R. acuta, R. americana, R. araucariae, R. arctica, R. armeniaca, R. aurantiaca, R. auriculariae, R. bacarum, R. benthica, R. biourgei, R. bogoriensis, R. bronchialis, R. buffonii, R. calyptogenae, R. chungnamensis, R. cladiensis, R. corallina, R. cresolica, R. crocea, R. cycloclastica, R. dairenensis, R. diffluens, R. evergladiensis, R. ferulica, R. foliorum, R. fragaria, R. fujisanensis, R. futronensis, R. gelatinosa, R. glacialis, R. glutinis, R. gracilis, R. graminis, R. grinbergsii, R. himalayensis, R. hinnulea, R. histolytica, R. hylophila, R. incarnata, R. ingeniosa, R. javanica, R. koishikawensis, R. lactosa, R. lamellibrachiae, R. laryngis, R. lignophila, R. lini, R. longissima, R. ludwigii, R. lysinophila, R. marina, R. martyniae-fragantis, R. matritensis, R. meli, R. minuta, R. mucilaginosa, R. nitens, R. nothofagi, R. oryzae, R. pacifica, R. pallida, R. peneaus, R. philyla, R. phylloplana, R. pilatii, R. pilimanae, R. pinicola, R. plicata, R. polymorpha, R. psychrophenolica, R. psychrophila, R. pustula, R. retinophila, R. rosacea, R. rosulata, R. rubefaciens, R. rubella, R. rubescens, R. rubra, R. rubrorugosa, R. rufula, R. rutila, R. sanguinea, R. sanniei, R. sartoryi, R. silvestris, R. simplex, R. sinensis, R. slooffiae, R. sonckii, R. straminea, R. subericola, R. suganii, R. taiwanensis, R. taiwaniana, R. terpenoidalis, R. terrea, R. texensis, R. tokyoensis, R. ulzamae, R. vanillica, R. vuilleminii, R. yarrowii, R. yunnanensis* and *R. zsoltii.* Suitable examples of *Phaffia* species include *P. rhodozyma.* Suitable examples of *Sporobolomyces* species include *S*. *alborubescens, S*. *bannaensis, S. beijingensis, S. bischofiae, S. clavatus, S. coprosmae, S. coprosmicola, S. corallinus, S. dimmenae, S. dracophylli, S. elongatus, S. gracilis, S. inositophilus, S. johnsonii, S*. *koalae, S*. *magnisporus, S. novozealandicus, S. odorus, S. patagonicus, S. productus, S*. *roseus, S. sasicola, S. shibatanus, S. singularis, S. subbrunneus, S. symmetricus, S. syzygii, S. taupoensis, S*. *tsugae, S. xanthus* and *S. yunnanensis.* Suitable examples of *Pachysolen* and *Moniliella* species include *P. tannophilus* and *M. pollinis,* respectively. Still other examples of non-conventional yeasts herein include *Pseudozyma* species (e.g., *S. antarctica*), *Thodotorula* species (e.g., *T. bogoriensis*), *Wickerhamiella* species (e.g., *W. domercqiae*), and *Starmerella* species (e.g., *S. bombicola*)*.*

*Yarrowia lipolytica* is preferred in certain embodiments disclosed herein. Examples of suitable *Y*. *lipolytica* include the following isolates available from the American Type Culture Collection (ATCC, Manassas, VA): strain designations ATCC #20362, #8862, #8661, #8662, #9773, #15586, #16617, #16618, #18942, #18943, #18944, #18945, #20114, #20177, #20182, #20225, #20226, #20228, #20327, #20255, #20287, #20297, #20315, #20320, #20324, #20336, #20341, #20346, #20348, #20363, #20364, #20372, #20373, #20383, #20390, #20400, #20460, #20461, #20462, #20496, #20510, #20628, #20688, #20774, #20775, #20776, #20777, #20778, #20779, #20780, #20781, #20794, #20795, #20875, #20241, #20422, #20423, #32338, #32339, #32340, #32341, #34342, #32343, #32935, #34017, #34018, #34088, #34922, #34922, #38295, #42281, #44601, #46025, #46026, #46027, #46028, #46067, #46068, #46069, #46070, #46330, #46482, #46483, #46484, #46436, #60594, #62385, #64042, #74234, #76598, #76861, #76862, #76982, #90716, #90811, #90812, #90813, #90814, #90903, #90904, #90905, #96028, #201241, #201242, #201243, #201244, #201245, #201246, #201247, #201249, and/or #201847.

A fungal cell herein can be a yeast (e.g., as described above) or of any other fungal type such as a filamentous fungus. For instance, a fungus herein can be a Basidiomycetes, Zygomycetes, Chytridiomycetes, or Ascomycetes fungus. Examples of filamentous fungi herein include those of the genera *Trichoderma, Chrysosporium, Thielavia, Neurospora* (e.g., *N. crassa, N. sitophila*), *Cryphonectria* (e.g., *C*. *parasitica*), *Aureobasidium* (e.g., *A. pullulans*), *Filibasidium, Piromyces, Cryplococcus, Acremonium, Tolypocladium, Scytalidium, Schizophyllum, Sporotrichum, Penicillium* (e.g., *P. bilaiae, P. camemberti, P. candidum, P. chrysogenum, P. expansum, P. funiculosum, P. glaucum, P. marneffei, P. roqueforti, P. verrucosum, P. viridicatum* ), *Gibberella* (e.g., *G. acuminata, G*. *avenacea, G. baccata, G. circinata, G. cyanogena, G. fujikuroi, G. intricans, G. pulicaris, G. stilboides, G. tricincta, G*. *zeae*), *Myceliophthora, Mucor* (e.g., *M. rouxii, M. circinelloides*), *Aspergillus* (e.g., *A. niger, A. oryzae, A. nidulans, A. flavus, A. lentulus, A. terreus, A. clavatus, A. fumigatus), Fusarium* (e.g., *F. graminearum, F. oxysporum, F. bubigenum, F. solani, F. oxysporum, F. verticillioides, F. proliferatum, F. venenatum*), and *Humicola,* and anamorphs and teleomorphs thereof. The genus and species of fungi herein can be defined, if desired, by morphology as disclosed in Barnett and Hunter (Illustrated Genera of Imperfect Fungi, 3rd Edition, Burgess Publishing Company, 1972). A fungus can optionally be characterized as a pest/pathogen of a plant or animal (e.g., human) in certain embodiments.

*Trichoderma* species in certain aspects herein include *T. aggressivum, T. amazonicum, T. asperellum, T. atroviride, T. aureoviride, T. austrokoningii, T. brevicompactum, T. candidum, T. caribbaeum, T. catoptron, T. cremeum, T. ceramicum, T. cerinum, T. chlorosporum, T. chromospermum, T. cinnamomeum, T. citrinoviride, T. crassum, T. cremeum, T. dingleyeae, T. dorotheae, T. effusum, T. erinaceum, T. estonicum, T. fertile, T. gelatinosus, T. ghanense, T. hamatum, T. harzianum, T. helicum, T. intricatum, T. konilangbra, T. koningii, T. koningiopsis, T. longibrachiatum, T. longipile, T. minutisporum, T. oblongisporum, T. ovalisporum, T. petersenii, T. phyllostahydis, T. piluliferum, T. pleuroticola, T. pleurotum, T. polysporum, T. pseudokoningii, T. pubescens, T. reesei, T. rogersonii, T. rossicum, T. saturnisporum, T. sinensis, T. sinuosum, T. spirale, T. stramineum, T. strigosum, T. stromaticum, T. surrotundum, T. taiwanense, T. thailandicum, T. thelephoricolum, T. theobromicola, T. tomentosum, T. velutinum, T. virens, T. viride* and *T. viridescens. A Trichoderma* species herein can be cultivated and/or manipulated as described in Trichoderma: Biology and Applications (P.K. Mukherjee et al., Eds., CABI, Oxfordshire, UK, 2013), for example.

A microbial cell in certain embodiments is an algal cell. For example, an algal cell can be from any of the following: Chlorophyta (green algae), Rhodophyta (red algae), Phaeophyceae (brown algae), Bacillariophycaeae (diatoms), and Dinoflagellata (dinoflagellates). An algal cell can be of a microalgae (e.g., phytoplankton, microphytes, or planktonic algae) or macroalgae (kelp, seaweed) in other aspects. As further examples, an algal cell herein can be a *Porphyra* (purple laver), *Palmaria* species such as *P. palmata* (dulse), *Arthrospira* species such as *A. platensis* (*spirulina*), *Chlorella* (e.g., *C. protothecoides*), *a Chondrus* species such as *C. crispus* (Irish moss), *Aphanizomenon, Sargassum, Cochayuyo, Botryococcus* (e.g., *B. braunii*), *Dunaliella* (e.g., D. *tertiolecta*), *Gracilaria, Pleurochrysis* (e.g., *P. carterae*), *Ankistrodesmus, Cyclotella, Hantzschia, Nannochloris, Nannochloropsis, Nitzschia, Phaeodactylum* (e.g., *P. tricomutum*), *Scenedesmus, Stichococcus, Tetraselmis* (e.g., *T. suecica*), *Thalassiosira* (e.g., *T. pseudonana*), *Crypthecodinium* (e.g., *C. cohnii*), *Neochloris* (e.g., *N. oleoabundans*), or *Schiochytrium.* An algal species herein can be cultivated and/or manipulated as described in Thompson (Algal Cell Culture. Encyclopedia of Life Support System (EOLSS), Biotechnology Vol 1, available at eolss.net/sample-chapters internet site), for example.

A protist cell herein can be selected from the class Ciliata (e.g., the genera *Tetrahymena, Paramecium, Colpidium, Colpoda, Glaucoma, Platyophrya, Vorticella, Potomacus, Pseudocohnilembus, Euplotes, Engelmaniella,* and *Stylonichia*), the subphylum Mastigophora (flagellates), the class Phytomastigophorea (e.g., the genera *Euglena, Astasia, Haematococcus,* and *Crypthecodinium*)*,* the class Zoomastigophorea, the superclass Rhizopoda, the class Lobosea (e.g., the genus *Amoeba*)*,* and the class Eumycetozoea (e.g., the genera *Dictyostelium* and *Physarum*), for example. Certain protist species herein can be cultivated and/or manipulated as described in ATCC^{®} Protistology Culture Guide: tips and techniques for propagating protozoa and algae (2013, available at American Type Culture Collection internet site), for example. A protist can optionally be characterized as a pest/pathogen of a plant or animal (e.g., human) in certain embodiments.

A bacterial cell in certain embodiments can be those in the form of cocci, bacilli, spirochetes, spheroplasts, protoplasts, etc. Other non-limiting examples of bacteria include those that are Gram-negative and Gram-positive. Still other non-limiting examples of bacteria include those of the genera *Salmonella* (e.g., *S. typhi, S. enteritidis*), *Shigella* (e.g., *S. dysenteriae*), *Escherichia* (e.g., *E. coli*), *Enterobacter, Serratia, Proteus, Yersinia, Citrobacter, Edwardsiella, Providencia, Klebsiella, Hafnia, Ewingella, Kluyvera, Morganella, Planococcus, Stomatococcus, Micrococcus, Staphylococcus* (e.g., *S*. *aureus, S. epidermidis), Vibrio* (e.g., *V. cholerae), Aeromonas, Plessiomonas, Haemophilus* (e.g., *H. influenzae*), *Actinobacillus, Pasteurella, Mycoplasma* (e.g., *M. pneumonia*), *Ureaplasma, Rickettsia, Coxiella, Rochalimaea, Ehrlichia, Streptococcus* (e.g., *S. pyogenes, S. mutans, S. pneumoniae), Enterococcus* (e.g., *E. faecalis*), *Aerococcus, Gemella, Lactococcus* (e.g., *L. lactis*), *Leuconostoc* (e.g., *L. mesenteroides*), *Pedicoccus, Bacillus* (e.g., B. *cereus, B. subtilis, B. thuringiensis*), *Corynebacterium* (e.g., *C. diphtheriae*), *Arcanobacterium, Actinomyces, Rhodococcus, Listeria* (e.g., *L. monocytogenes*), *Erysipelothrix, Gardnerella, Neisseria* (e.g., *N. meningitidis, N. gonorrhoeae*), *Campylobacter, Arcobacter, Wolinella, Helicobacter* (e.g., *H. pylori*), *Achromobacter, Acinetobacter, Agrobacterium* (e.g., *A. tumefaciens*), *Alcaligenes, Chryseomonas, Comamonas, Eikenella, Flavimonas, Flavobacterium, Moraxella, Oligella, Pseudomonas* (e.g., *P. aeruginosa*), *Shewanella, Weeksella, Xanthomonas, Bordetella, Franciesella, Brucella, Legionella, Afipia, Bartonella, Calymmatobacterium, Cardiobacterium, Streptobacillus, Spirillum, Peptostreptococcus, Peptococcus, Sarcinia, Coprococcus, Ruminococcus, Propionibacterium, Mobiluncus, Bifidobacterium, Eubacterium, Lactobacillus* (e.g., L. *lactis, L. acidophilus*), *Rothia, Clostridium* (e.g., *C. botulinum, C. perfringens*), *Bacteroides, Porphyromonas, Prevotella, Fusobacterium, Bilophila, Leptotrichia, Wolinella, Acidaminococcus, Megasphaera, Veilonella, Norcardia, Actinomadura, Norcardiopsis, Streptomyces, Micropolysporas, Thermoactinomycetes, Mycobacterium* (e.g., *M. tuberculosis, M. bovis, M. leprae*), *Treponema, Borrelia* (e.g., *B. burgdorferi), Leptospira, and Chlamydiae.* A bacteria can optionally be characterized as a pest/pathogen of a plant or animal (e.g., human) in certain embodiments. Bacteria can be comprised in a mixed microbial population (e.g., containing other bacteria, or containing yeast and/or other bacteria) in certain embodiments.

An archaeal cell in certain embodiments can be from any Archaeal phylum, such as Euryarchaeota, Crenarchaeota, Nanoarchaeota, Korarchaeota, Aigarchaeota, or Thaumarchaeota. Archaeal cells herein can be extremophilic (e.g., able to grow and/or thrive in physically or geochemically extreme conditions that are detrimental to most life), for example. Some examples of extremophilic archaea include those that are thermophilic (e.g., can grow at temperatures between 45-122 °C), hyperthermophilic (e.g., can grow at temperatures between 80-122 °C), acidophilic (e.g., can grow at pH levels of 3 or below), alkaliphilic (e.g., can grow at pH levels of 9 or above), and/or halophilic (e.g., can grow in high salt concentrations [e.g., 20-30% NaCl]). Examples of archaeal species include those of the genera *Halobacterium* (e.g., *H. volcanii*), *Sulfolobus* (e.g., *S. solfataricus, S. acidocaldarius), Thermococcus* (e.g., *T. alcaliphilus, T. celer, T. chitonophagus, T. gammatolerans, T. hydrothermalis, T. kodakarensis, T. litoralis, T. peptonophilus, T. profundus, T. stetteri*), *Methanocaldococcus* (e.g., *M. thermolithotrophicus, M. jannaschii), Methanococcus* (e.g., *M. maripaludis*), *Methanothermobacter* (e.g., *M. marburgensis, M. thermautotrophicus*), *Archaeoglobus* (e.g., *A. fulgidus*), *Nitrosopumilus* (e.g., *N*. *maritimus*), *Metallosphaera* (e.g., *M. sedula*)*, Ferroplasma, Thermoplasma, Methanobrevibacter* (e.g., *M. smithii),* and *Methanosphaera* (e.g., *M. stadtmanae).*

Examples of insect cells herein include *Spodoptera frugiperda* cells, *Trichoplusia ni* cells, *Bombyx mori* cells and the like. *S. frugiperda* cells include Sf9 and Sf21, for instance. *T. ni* ovary cells include HIGH FIVE cells (alias BTI-TN-5B1-4, manufactured by Invitrogen), for example. B. mori cells include N4, for example. Certain insect cells herein can be cultivated and/or manipulated as described in Growth and Maintenance of Insect cell lines (2010, Invitrogen, Manual part no. 25-0127, MAN0000030), for example. In other aspects, an insect cell can be a cell of a plant pest/pathogen such as an armyworm, black cutworm, corn earworm, corn flea beetle, corn leaf aphid, corn root aphid, European corn borer, fall armyworm, granulate cutworm, Japanese beetle, lesser cornstalk borer, maize billbug, melanotus communis, seedcorn maggot, sod webworms, sorghum midge, sorghum webworm, southern corn billbug, southern corn rootworm, southern cornstalk borer, southern potato wireworm, spider mite, stalk borer, sugarcane beetle, tobacco wireworm, white grub, aphid, boll weevil, bollworm complex, cabbage looper , tarnished plant bug, thrip, two spotted spider mite, yellow striped armyworm, alfalfa weevil, clover leaf weevil, clover root curculio, fall armyworm, grasshopper, meadow spittlebug, pea aphid, potato leafhopper, sod webworm, variegated cutworm, lesser cornstalk borer, tobacco thrip, wireworm, cereal leaf beetle, chinch bug, English grain aphid, greenbug, hessian fly, bean leaf beetle, beet armyworm, blister beetle, grape colaspis, green cloverworm, Mexican bean beetle, soybean looper, soybean stem borer, stink bug, three-cornered alfalfa hopper, velvetbean caterpillar, budworm, cabbage looper, cutworm, green june beetle, green peach aphid, hornworm, potato tuberworm, southern mole cricket, suckfly, tobacco flea beetle, vegetable weevil, or whitefringed beetle. Alternatively, an insect cell can be a cell of a pest/pathogen of an animal (e.g., human).

A nematode cell, for example, can be of a nematode from any of the following genera: *Meloidogyne* (root-knot nematode), *Pratylenchus* (lesion nematode), *Heterodera* (cyst nematode), *Globodera* (cyst nematode), *Ditylenchus* (stem and bulb nematode), *Tylenchulus* (citrus nematode), *Xiphinema* (dagger nematode), *Radopholus* (burrowing nematode), *Rotylenchulus* (reniform nematode), *Helicotylenchus* (spiral nematode), or *Belonolaimus* (sting nematode). A nematode can optionally be characterized as a pest/pathogen of a plant or animal (e.g., human) in certain embodiments. A nematode can be *C*. *elegans* in other aspects.

A fish cell herein can be any of those as disclosed in U.S. Patent Nos. 7408095 and 7217564, and Tissue Culture of Fish Cell Lines (T. Ott, NWFHS Laboratory Procedures Manual - Second Edition, Chapter 10, 2004), for example. These references also disclose information regarding cultivating and/or manipulating fish cells. Non-limiting examples of fish cells can be from a teleost such as zebrafish, medaka, Giant rerio, or puffer fish.

Mammalian cells in certain embodiments can be human, non-human primate (e.g., monkey, ape), rodent (e.g., mouse, rat, hamster, guinea pig), rabbit, dog, cat, cow, pig, horse, goat, or sheep cells. Other examples of mammalian cells herein include primary epithelial cells (e.g., keratinocytes, cervical epithelial cells, bronchial epithelial cells, tracheal epithelial cells, kidney epithelial cells, retinal epithelial cells); established cell lines (e.g., 293 embryonic kidney cells, HeLa cervical epithelial cells, PER-C6 retinal cells, MDBK, CRFK, MDCK, CHO, BeWo, Chang cells, Detroit 562, Hep-2, KB, LS 180, LS 174T, NCI-H-548, RPMI 2650, SW-13, T24, WI-28 VA13, 2RA, WISH, BS-C-I, LLC-MK2, Clone M-3, RAG, TCMK-1, LLC-PK1, PK-15, GH1, GH3, L2, LLC-RC 256, MH1C1, XC, MDOK, VSW, TH-I, B1 cells); any epithelial, mesenchymal (e.g., fibroblast), neural, or muscular cell from any tissue or organ (e.g., skin, heart; liver; kidney; colon; intestine; esophagus; stomach; neural tissue such as brain or spinal cord; lung; vascular tissue; lymphoid tissue such as lymph gland, adenoid, tonsil, bone marrow, or blood; spleen); and fibroblast or fibroblast-like cell lines (e.g., TRG-2, IMR-33, Don cells, GHK-21, citrullinemia cells, Dempsey cells, Detroit 551, Detroit 510, Detroit 525, Detroit 529, Detroit 532, Detroit 539, Detroit 548, Detroit 573, HEL 299, IMR-90, MRC-5, WI-38, WI-26, MiCl1, CV-1, COS-1, COS-3, COS-7, Vero, DBS-FrhL-2, BALB/3T3, F9, SV-T2, M-MSV-BALB/3T3, K-BALB, BLO-11, NOR-10, C3H/IOTI/2, HSDM1C3, KLN205, McCoy cells, Mouse L cells, SCC-PSA1, Swiss/3T3 cells, Indian muntjac cells, SIRC, Jensen cells). Methods of culturing and manipulating mammalian cells lines are known in the art.

The term "plant" refers to whole plants, plant organs, plant tissues, seeds, plant cells, seeds and progeny of the same. Plant cells include, without limitation, cells from seeds, suspension cultures, embryos, meristematic regions, callus tissue, leaves, roots, shoots, gametophytes, sporophytes, pollen and microspores. Plant parts include differentiated and undifferentiated tissues including, but not limited to roots, stems, shoots, leaves, pollens, seeds, tumor tissue and various forms of cells and culture (e.g., single cells, protoplasts, embryos, and callus tissue). The plant tissue may be in plant or in a plant organ, tissue or cell culture. The term "plant organ" refers to plant tissue or a group of tissues that constitute a morphologically and functionally distinct part of a plant. The term "genome" refers to the entire complement of genetic material (genes and non-coding sequences) that is present in each cell of an organism, or virus or organelle; and/or a complete set of chromosomes inherited as a (haploid) unit from one parent. "Progeny" comprises any subsequent generation of a plant.

A transgenic plant includes, for example, a plant which comprises within its genome a heterologous polynucleotide introduced by a transformation step. The heterologous polynucleotide can be stably integrated within the genome such that the polynucleotide is passed on to successive generations. The heterologous polynucleotide may be integrated into the genome alone or as part of a recombinant DNA construct. A transgenic plant can also comprise more than one heterologous polynucleotide within its genome. Each heterologous polynucleotide may confer a different trait to the transgenic plant. A heterologous polynucleotide can include a sequence that originates from a foreign species, or, if from the same species, can be substantially modified from its native form. Transgenic can include any cell, cell line, callus, tissue, plant part or plant, the genotype of which has been altered by the presence of heterologous nucleic acid including those transgenics initially so altered as well as those created by sexual crosses or asexual propagation from the initial transgenic. The alterations of the genome (chromosomal or extra-chromosomal) by conventional plant breeding methods, by the genome editing procedure described herein that does not result in an insertion of a foreign polynucleotide, or by naturally occurring events such as random cross-fertilization, non-recombinant viral infection, non-recombinant bacterial transformation, non-recombinant transposition, or spontaneous mutation are not intended to be regarded as transgenic.

A fertile plant is a plant that produces viable male and female gametes and is self-fertile. Such a self-fertile plant can produce a progeny plant without the contribution from any other plant of a gamete and the genetic material contained therein. Male-sterile plants include plants that do not produce male gametes that are viable or otherwise capable of fertilization. Female-sterile plants include plants that do not produce female gametes that are viable or otherwise capable of fertilization. It is recognized that male-sterile and female-sterile plants can be female-fertile and male-fertile, respectively. It is further recognized that a male-fertile (but female-sterile) plant can produce viable progeny when crossed with a female-fertile plant and that a female-fertile (but male-sterile) plant can produce viable progeny when crossed with a male-fertile plant.

Any plant can be used, including monocot and dicot plants. Examples of monocot plants that can be used include, but are not limited to, corn (*Zea mays),* rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet (*Eleusine coracana*)), wheat (*Triticum aestivum*), sugarcane (*Saccharum spp*.), oats (*Avena*), barley (*Hordeum*), switchgrass (*Panicum virgatum*), pineapple (*Ananas comosus*), banana (*Musa spp*.), palm, ornamentals, turfgrasses, and other grasses. Examples of dicot plants that can be used include, but are not limited to, soybean (*Glycine max*), canola (*Brassica napus* and *B*. *campestris*), alfalfa (*Medicago sativa*)*,* tobacco (*Nicotiana tabacum*), *Arabidopsis (Arabidopsis thaliana),* sunflower (*Helianthus annuus*), cotton (*Gossypium arboreum*), and peanut (*Arachis hypogaea*)*,* tomato (*Solanum lycopersicum*), potato (*Solanum tuberosum*) *etc.*

The term "dicot" refers to the subclass of angiosperm plants also knows as "dicotyledoneae" and includes reference to whole plants, plant organs (e.g., leaves, stems, roots, etc.), seeds, plant cells, and progeny of the same. Plant cell, as used herein includes, without limitation, seeds, suspension cultures, embryos, meristematic regions, callus tissue, leaves, roots, shoots, gametophytes, sporophytes, pollen, and microspores.

The terms "5'-cap" and "7-methylguanylate (m⁷G) cap" are used interchangeably herein. A 7-methylguanylate residue is located on the 5' terminus of RNA transcribed by RNA polymerase II (Pol II) in eukaryotes. A capped RNA herein has a 5'-cap, whereas an uncapped RNA does not have such a cap.

The terminology "uncapped", "not having a 5'-cap", and the like are used interchangeably herein to refer to RNA lacking a 5'-cap and optionally having, for example, a 5'-hydroxyl group instead of a 5'-cap. Uncapped RNA can better accumulate in the nucleus following transcription, since 5'-capped RNA is subject to nuclear export.

The terms "ribozyme", "ribonucleic acid enzyme" and "self-cleaving ribozyme" are used interchangeably herein. A ribozyme refers to one or more RNA sequences that form secondary, tertiary, and/or quaternary structure(s) that can cleave RNA at a specific site, particularly at a cis-site relative to the ribozyme sequence (i.e., autocatalytic, or self-cleaving). The general nature of ribozyme nucleolytic activity has been described (e.g., Lilley, Biochem. Soc. Trans. 39:641-646). A "hammerhead ribozyme" (HHR) may comprise a small catalytic RNA motif made up of three base-paired stems and a core of highly conserved, non-complementary nucleotides that are involved in catalysis. Pley et al. (Nature 372:68-74) and Hammann et al. (RNA 18:871-885) disclose hammerhead ribozyme structure and activity. A hammerhead ribozyme may comprise a "minimal hammerhead" sequence as disclosed by Scott et al. (Cell 81:991-1002), for example.

The term "increased" as used herein may refer to a quantity or activity that is at least about 1%, 2%, 3%, 4%, 5%, 6%, 7%, 8%, 9%, 10%, 11%, 12%, 13%, 14%, 15%, 16%, 17%, 18%, 19%, 20%, 50%, 100%, or 200% more than the quantity or activity for which the increased quantity or activity is being compared. The terms "increased", "elevated", "enhanced", "greater than", and "improved" are used interchangeably herein. The term "increased" can be used to characterize the expression of a polynucleotide encoding a protein, for example, where "increased expression" can also mean "over-expression".

A variety of methods are available to identify those cells having an altered genome at or near a target site without using a screenable marker phenotype. Such methods can be viewed as directly analyzing a target sequence to detect any change in the target sequence, including but not limited to PCR methods, sequencing methods, nuclease digestion, Southern blots, and any combination thereof.

Standard DNA isolation, purification, molecular cloning, vector construction, and verification/characterization methods are well established, see, for example Sambrook et al., (1989) Molecular Cloning: A Laboratory Manual, (Cold Spring Harbor Laboratory Press, NY). Vectors and constructs include circular plasmids, and linear polynucleotides, comprising a polynucleotide of interest and optionally other components including linkers, adapters, regulatory or analysis. In some examples a recognition site and/or target site can be contained within an intron, coding sequence, 5' UTRs, 3' UTRs, and/or regulatory regions.

The meaning of abbreviations is as follows: "sec" means second(s), "min" means minute(s), "h" means hour(s), "d" means day(s), "µL" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s) and "kb" means kilobase(s).

### EXAMPLES

In the following Examples, unless otherwise stated, parts and percentages are by weight and degrees are Celsius. It should be understood that these Examples are given by way of illustration only.

### EXAMPLE 1

### Csy4 or NLS-Csy4 is functional in Yarrowia

The present Example describes cloning of a Csy4 (also known as Cas6) encoding gene into a Cas9 expression plasmid also comprising a DNA recombinant DNA encoding a CAN1 targeting sgRNA flanked by 28-nucleotide Csy4 recognition sites, for CAN1 gene inactivation in *Yarrowia.* Two different recombinant constructs were made wherein enabling Csy4 expression in the presence or absence of a N-terminal nuclear localization sequence (NLS).

pYRH286 expressed a NLS-Cas9 endonuclease (SEQ ID NO: 1) under a FBA1 promoter (SEQ ID NO: 2) and a *Yarrowia lipolytica* codon-optimized gene for Csy4 (SEQ ID NO: 3) expression under a FBA1 promoter (SEQ ID NO: 2).

pYRH290 was based on pYRH286 and additionally contained an DNA fragment (SEQ ID NO: 4) expressing the 28-nucleotide (nt) Csy4 endonuclease recognition sequence (SEQ ID:5) flanked pre-sgRNA (SEQ ID NO:6) targeting a CAN1 target sequence (SEQ ID NO:7) under a TDH1 promoter (SEQ ID NO: 8) which is a RNA polymerase II (Pol-II) promoter (FIGURE 1B).

pYRH319 was based on pYRH290, wherein the gene encoding Csy4 was replaced by a gene encoding NLS fusion to Yarrowia codon optimized P. aeruginosa Csy4(SEQ ID NO: 9) where NLS is Simian virus 40 (SV40) monopartite amino terminal NLS (SEQ ID NO: 10).

pYRH327 was based on pYRH290, wherein the gene encoding Csy4 was deleted. Therefore, pYRH327 expressed Cas9 and the 28-nucleotide (nt) Csy4 endonuclease recognition sequence (SEQ ID: 5) flanked pre-sgRNA (SEQ ID NO: 6).

All the vectors contained an incomplete Autonomously Replicating Sequence ARS18 (SEQ ID NO: 11) that lacked 3'-end 27bp of the full length ARS18 (SEQ ID: 12).

A Ura-minus derivative (Y2224) of *Yarrowia* strain ATCC20362 was transformed with the plasmids, and transformants were selected on CM plates lacking uracil. Transformants grown on the selective plates were replica-plated to CM plates containing canavanine to select for *can1* mutants. Colonies grown on the CM plates containing canavanine has been counted, and the mutations at the target site have been confirmed by sequencing.

Table 2 shows that colonies transformed with an NLS-Cas9, a gRNA flanked by Csy4 sites, and a NLS-Csy4 (see pYRH319) were about 23% more effective in generating targeted mutations in CAN1 than colonies transformed with an NLS-Cas9, a gRNA flanked by Csy4 sites, and a Csy4 (pYRH290) . Interestingly, transformants that expressed a NLS-Cas9 and a gRNA flanked by Csy4 sites but did not express CSY4 (pYRH327) also produced targeted mutants.

**Table 2**

| **Analysis of pYRH286, pYRH290, pYRH319, and pYRH327 transformants. Results are from three experiments** | | | | |
|---|---|---|---|---|
| | Cas9 | Csy4 | gRNA flaked by Csy4 sites | *CAN1* mutants (% of transformants) |
| pYRH286 | NLS-Cas9 | Csy4 | No | 0 |
| pYRH290 | NLS-Cas9 | Csy4 | Yes | 35 +/- 5 |
| pYRH319 | NLS-Cas9 | NLS-Csy4 | Yes | 43 +/- 3.5 |
| pYRH327 | NLS-Cas9 | No | Yes | 22 +/- 1 |

Next, experiments were performed to determine if functional guide RNA's can be directly expressed by an RNA polymerase II promoter.

### EXAMPLE 2

### gRNA expressed under RNA polymerase II (Pol-II) is functional in Yarrowia

The present Example describes the expression of *CAN1* targeting sgRNA in *Yarrowia* by a recombinant DNA construct lacking 28-nt Csy4 recognition sites flanking the DNA encoding the single guide RNA operably linked to a RNA polymerase II promoter, in the presence or absence of a terminator sequence (Figure 1 A). The effectiveness of these recombinant DNA construct were compared to recombinant DNA constructs having 28-nt Csy4 recognition sites flanking the DNA encoding the single guide RNA (Figure 1 B) Cas9 was co-expressed on a plasmid for CAN1 gene inactivation in *Yarrowia.*

Plasmids pYRH376, pYRH378, pYRH379, and pYRH380 were constructed to test whether RNA polymerase II promoters can produce functional gRNAs. All plasmids used the same backbone plasmid (pRF291) that expresses NLS-Cas9 endonuclease (SEQ ID NO: 1) under a FBA1 promoter (SEQ ID NO: 2). All plasmids contained the full length ARS18 (SEQ ID: 12).

pYRH376 expresses the 28-nt Csy4 endonuclease recognition sequence (SEQ ID:5) flanked pre-sgRNA (SEQ ID NO:6) targeting a *CAN1* target sequence (SEQ ID NO:7) under *TDH1* promoter (SEQ ID NO: 8) which is RNA polymerase II promoter.

pYRH378 expresses pre-sgRNA (SEQ ID NO:6) targeting a CAN1 target sequence (SEQ ID NO:7) under *TDH1* promoter (SEQ ID NO: 8).

pYRH379 expresses pre-sgRNA (SEQ ID NO:6) targeting a CAN1 target sequence (SEQ ID NO:7) under *TDH1* promoter (SEQ ID NO: 8) and also with *TDH1* terminator sequence (SEQ ID NO: 13) at the 3' of pre-sgRNA.

pYRH380 expresses the 28-nt Csy4 endonuclease recognition sequence (SEQ ID:5) flanked pre-sgRNA (SEQ ID NO:6) targeting a *CAN1* target sequence (SEQ ID NO:7) under *FBA1* promoter (SEQ ID NO: 2) which is RNA polymerase II promoter.

A Ura-minus derivative (Y2224) of *Yarrowia* strain ATCC20362 was transformed with the plasmids, and transformants were selected on CM plates lacking uracil. Transformants grown on the selective plates were replica-plated to CM plates containing canavanine to select for *can1* mutants. Colonies grown on the CM plates containing canavanine has been counted to calculated the mutation frequency by comparing with the total number of transformants.

As shown in Figure 2, the gRNA expressed under RNA polymerase II promoters were functional and produced canavanine resistance mutants at about 70% of all transformants, regardless of the presence or absence of the 28-nt Csy4 endonuclease recognition sequence 5'- and 3' of the gRNA.

### EXAMPLE 3

### Cas9 expression plasmid and construction of RNA Polymerase II (Pol-II) gRNA expression cassettes

This example discusses the construction of a *Y*. *lipolytica* plasmid for the constitutive expression of *S*. *pyogenes* Cas9 protein, the construction of RNA polymerase II gRNA expression cassettes, and the insertion of these expression cassettes into the Cas9 expression plasmid to create a single *Y*. *lipolytica* plasmid constitutively expressing both Cas9 and a gRNA targeting a *Y*. *lipolytica* chromosomal sequence.

In order to test a sgRNA/Cas endonuclease system in *Yarrowia,* the Cas9 gene from *Streptococcus pyrogenes* M1 GAS (SF370 (SEQ ID NO: 1) was *Yarrowia* codon optimized per standard techniques known in the art (SEQ ID NO: 14). In order to localize the Cas9 protein to the nucleus of the cells, *Simian virus* 40 (SV40) monopartite (PKKKRKV, SEQ ID NO: 15) nuclear localization signal was incorporated at the carboxy terminus of the Cas9 protein. The Yarrowia codon optimized Cas9 gene was fused to a Yarrowia constitutive promoter, FBA1 (SEQ ID NO: 16), by standard molecular biology techniques. An example of a Yarrowia codon optimized Cas9 expression cassette (SEQ ID NO: 17) contains the FBA1 promoter, the Yarrowia optimized Cas9-NLS fusion, and the The Cas9 expression cassette was cloned into the plasmid pZuf and the new construct called pZufCas9 (SEQ ID NO 18).

In order to create RNA polymerase II transcribed gRNA expression cassettes (Figure3A-3C) different promoters (*FBA1* or *TEF1)* were combined at their transcriptional start site (TSS) or at the end of the 5' untranslated region (UTR) with a DNA fragment encoding the gRNA targeting the Can1-1 site. The 3' side of the gRNA was either fused with a RNA polymerase II terminator (*ACT1*) or with the DNA encoding the HDV ribozyme and then the *ACT1* terminator. These constructs test the optimal fusion of the promoter with the gRNA and if the presence of the HDV ribozyme (which will autocatalytically remove itself and any transcribed terminator sequence from the 3' end of the gRNA) allows gene targeting by the *S. pyogenes* Cas9 protein.

The *TEF1_{TSS}* promoter fragment (SEQ ID NO: 19) was amplified from *Y. lipolytica* genomic DNA using standard PCR (gggttaattaaAGAGACCGGGTTGGCGGCGC (SEQ ID NO: 20), Forward and, gagggtgggtaatcgtttgattgaCAAGGAGAGAGAGAAA (SEQ ID NO: 21), Reverse). The forward primer adds a PacI restriction endonuclease site. The reserve primer adds the first 20nucleotides of the DNA encoding the Can1-1 gRNA. The *TEF1_{UTR}* promoter (SEQ ID NO: 22) was amplified from Y. *lipolytica* genomic DNA using standard PCR (gggttaattaaAGAGACCGGGTTGGCGGCGC (SEQ ID NO: 20), Forward and gagggtgggtaatcgtttgaTTTGAATGATTCTTATACTC (SEQ ID NO: 23), Reverse). The forward primer adds a PacI restriction site and the reverse primer adds the first 20 nucleotides of the DNA encoding the Can1-1 gRNA. The *FBA1_{TSS}* promoter fragment (SEQ ID NO: 24) was amplified from pZufCas9 (SEQ ID NO: 18) using standard PCR (gggttaattaagtttaaaccatcatctaagggcc (SEQ ID NO: 25), forward and gagggtgggtaatcgtttgatggcaaccgattgggagagc (SEQ ID NO: 26), reverse). The forward primers adds a PacI restriction endonuclease site and the reverse primer adds 20 nucleotides of the DNA encoding the Can1-1 gRNA. The *FBA1_{UTR}* promoter (SEQ ID NO: 27) was amplified from pZufCas9 (SEQ ID NO: 18) using standard PCR (gggttaattaagtttaaaccatcatctaagggcc (SEQ ID NO: 25), forward and gagggtgggtaatcgtttgaggtgtgatgtgtagtttaga (SEQ ID NO: 28), reverse). The forward primer adds a PacI endonuclease recognition site and the reverse primer adds 20 nucleotides of the DNA encoding the Can1-1 gRNA.

The *ACT1* terminator fragment (SEQ ID NO: 29) for fusion to the DNA encoding the Can1-1 gRNA was amplified from pFB23 (SEQ ID NO: 30) using standard PCR (accgagtcggtggtgcttttGGCCGCgtgtggtgattgct (SEQ ID NO: 31), forward and ggggatcgattggaagagatttcgaagcacg, (SEQ ID NO: 32) reverse). The forward primer adds the 3' most 20 nucleotides of the DNA encoding the Can1-1 gRNA and the reverse primer adds a Clal restriction endonuclease recognition site. The *ACT1* terminator (SEQ ID NO: 33) for fusion to the DNA encoding the 3' HDV flanked Can1-1 gRNA was amplified from pFB23 (SEQ ID NO: 30) using standard PCR (cttcggcatggcgaatgggaGGCCGCgtgtggtgattgct (SEQ ID NO: 34), forward and ggggatcgattggaagagatttcgaagcacg (SEQ ID NO: 32), reverse). The forward primer adds the 3' most 20 nucleotides of the DNA encoding the 3' HDV flanked Can1-1 gRNA and the reverse primer adds a ClaI site.

The DNA encoding the Can1-1 gRNA (SEQ ID NO: 35) was amplified for fusion to the *FBA1_{TSS}* fragment (SEQ ID NO: 24) and the *ACT1* terminator fragment (SEQ ID NO: 29) using standard PCR using pRF84 (SEQ ID NO: 36) (gctctcccaatcggttgccatcaaacgattacccaccctc (SEQ ID NO: 37), forward and agcaatcaccacacGCGGCCaaaagcaccaccgactcggt (SEQ ID NO: 38), reverse). The forward primer adds 20 nucleotides corresponding to the 3' most 20 nucleotides of the *FBA1_{TSS}* fragment and the reverse primer adds 20 nucleotides corresponding to the 5' most 20 nucleotides of the *ACT1* terminator fragment. The DNA encoding the 3' HDV flanked Can1-1 gRNA (SEQ ID NO: 39) was amplified from pRF84 (SEQ ID NO: 36) for fusion to the FBA1*_{TSS}* promoter (SEQ ID NO: 24) fragment and the *ACT1* terminator fragment (SEQ ID NO: 33) using standard PCR (gctctcccaatcggttgccatcaaacgattacccaccctc (SEQ ID NO: 37), forward and agcaatcaccacacGCGGCCtcccattcgccatgccgaag (SEQ ID NO: 40), reverse). The forward primer adds the 3' most 20 nucleotides of the *FBA1_{TSS}* fragment and the the reverse primer adds the 5' most 20 nucleotides of the *ACT1* terminator. The DNA encoding the Can1-1 gRNA (SEQ ID NO: 41) was amplified from pRF84 (SEQ ID NO: 36) for fusion to the *FBA1_{UTR}* promoter fragment (SEQ ID NO: 27) and the *ACT1* terminator fragment (SEQ ID NO: 29) using standard PCR (tctaaactacacatcacacctcaaacgattacccaccctc (SEQ ID NO: 42), forward and agcaatcaccacacGCGGCCaaaagcaccaccgactcggt (SEQ ID NO: 38), reverse). The forward primer adds the 3' most 20 nucleotides of the *FBA1_{UTR}* fragment and the reverse primer adds the 5' most 20 nucleotides of the *ACT1* terminator fragment. The DNA encoding the 3' HDV flanked Can1-1 gRNA (SEQ ID NO: 43) was amplified from pRF84 (SEQ ID NO: 36) for fusion to the *FBA1_{UTR}* fragment (SEQ ID NO: 27) and the *ACT1* terminator (SEQ ID NO: 33) fragment using standard PCR (tctaaactacacatcacacctcaaacgattacccaccctc (SEQ ID NO: 42), forward and agcaatcaccacacGCGGCCtcccattcgccatgccgaag (SEQ ID NO: 40), reverse). The forward primer adds the 3' most 20 nucleotides of the *FBA1_{UTR}* fragment and the reverse primer adds the 5' most 20 nucleotides of the *ACT1* terminator fragment. The DNA encoding the Can1-1 gRNA (SEQ ID NO: 44) was amplified from pRF84 (SEQ ID NO: 36) for fusion to the *TEF1_{TSS}* fragment (SEQ ID NO: 19) and the *ACT1* terminator (SEQ ID NO: 29) fragment using standard PCR (TTTCTCTCTCTCCTTGtcaatcaaacgattacccaccctc (SEQ ID NO: 45), forward and agcaatcaccacacGCGGCCaaaagcaccaccgactcggt (SEQ ID NO: 38), reverse). The forward primer adds the 3' most 20 nucleotides of the *TEF1_{TSS}* fragment and the reverse primer adds the 5' most 20 nucleotides of the *ACT1* terminator fragment. The 3' HDV ribozyme flanked Can1-1 gRNA (SEQ ID NO: 46) was amplified from pRF84 (SEQ ID NO: 36) for fusion to the *TEF1_{TSS}* fragment (SEQ ID NO: 19) and the *ACT1* terminator fragment (SEQ ID NO: 33) using standard PCR (TTTCTCTCTCTCCTTGtcaatcaaacgattacccaccctc SEQ ID NO: 44), forward and agcaatcaccacacGCGGCCtcccattcgccatgccgaag (SEQ ID NO: 40), reverse). The forward primer adds the 3' most 20 nucleotides of the *TEF1_{TSS}* fragment and the reverse primer adds the 5' most 20 nucleotides of the *ACT1* terminator. The DNA encoding the Can1-1 gRNA (SEQ ID NO: 47) was amplified from pRF84 (SEQ ID NO: 36) for fusion to the *TEF1_{UTR}* fragment (SEQ ID NO: 22) and the *ACT1* terminator fragment (SEQ ID NO: 29) using standard PCR (gagggtgggtaatcgtttgaTTTGAATGATTCTTATACTC (SEQ ID NO: 48), forward and agcaatcaccacacGCGGCCaaaagcaccaccgactcggt (SEQ ID NO: 38), reverse). The forward primer adds the 3' most 20 nucleotides of the *TEF1_{UTR}* fragment and the reverse primer adds the 5' most 20 nucleotides of the *ACT1* terminator fragment. The DNA encoding the 3' HDV flanked gRNA targeting (SEQ ID NO: 49) Can1-1 was amplified from pRF84 (SEQ ID NO: 36) for fusion with the *TEF1_{UTR}* fragment (SEQ ID NO: 22) and the *ACT1* terminator (SEQ ID NO: 33) fragment using standard PCR (gagggtgggtaatcgtttgaTTTGAATGATTCTTATACTC (SEQ ID NO: 48), forward and agcaatcaccacacGCGGCCtcccattcgccatgccgaag (SEQ ID NO: 40), reverse). The forward primer adds the 3' most 20 nucleotides of the *TEF1_{UTR}* fragment and the reverse primer adds the 5' most 20 nucleotides of the *ACT1* fragment.

Assembly of the promoter/gRNA/terminator fragments into RNA polymerase II expression cassettes was performed using synthesis from overlapping ends producing a single DNA molecule containing all three parts (Horton et al (2013) Biotechniques 54(3):129-133). A list of the parts combined to build specific constructs can be found in Table 3. The final constructs (Table 3) were digested with PacI/ClaI and cloned into the same sites of pZufCas9 (SEQ ID NO: 18).

**Table 3**

| **Parts used to build RNA polymerase II gRNA expression constructs** | | | | |
|---|---|---|---|---|
| **Expression Construct** | **Promoter** | **gRNA** | **Terminator** | **Plasmid** |
| *FBA1_{TSS}-*Can1-1-*ACT1* (SEQ ID NO: 50) | SEQ ID NO: 24 | SEQ ID NO: 35 | SEQ ID NO: 29 | pRF617 (SEQ ID NO: 61) |
| *FBA1_{TSS}*-Can1-1HDV-*ACT1* (SEQ ID NO: 51) | SEQ ID NO: 24 | SEQ ID NO: 39 | SEQ ID NO: 33 | pRF616 (SEQ ID NO: 62) |
| *FBA1_{UTR}*-Can1-1-*ACT1* (SEQ ID NO: 52) | SEQ ID NO: 27 | SEQ ID NO: 41 | SEQ ID NO: 29 | pRF619 (SEQ ID NO: 63) |
| *FBA1_{UTR}*-Can1-1HDV-*ACT1* (SEQ ID NO: 53) | SEQ ID NO: 27 | SEQ ID NO: 43 | SEQ ID NO: 33 | pRF618 (SEQ ID NO: 64) |
| *TEF1_{TSS}*-Can1-1-*ACT1* (SEQ ID NO: 54) | SEQ ID NO: 19 | SEQ ID NO: 44 | SEQ ID NO: 29 | pRF626 (SEQ ID NO: 65) |
| *TEF1_{TSS}*-Can1-1HDV-*ACT1* (SEQ ID NO: 55) | SEQ ID NO: 19 | SEQ ID NO: 46 | SEQ ID NO: 33 | pRF625 (SEQ ID NO: 66) |
| *TEF1_{UTR}*-Can1-1-*ACT1* (SEQ ID NO: 56) | SEQ ID NO: 22 | SEQ ID NO: 47 | SEQ ID NO: 29 | pRF623 (SEQ ID NO: 67) |
| *TEF1_{UTR}*-Can1-1HDV-*ACT1* (SEQ ID NO: 57) | SEQ ID NO: 22 | SEQ ID NO: 49 | SEQ ID NO: 33 | pRF621 (SEQ ID NO: 68) |

Presence and sequence of the RNA polymerase II gRNA expression cassettes was confirmed via sanger sequencing with primers HY009 (SEQ ID NO:58), HY010 (SEQ ID NO: 59), and ON476 (SEQ ID NO: 60). The plasmids containing each RNA Pol II expression construct (Table 2) were used to target the Can1-1 target site (SEQ ID NO: 61) in *Y*. *lipolytica.*

### EXAMPLE 4

### Targeting the Can1-1 target site with Cas9 and RNA Pol II expressed gRNA

In order to test if gRNA can be expressed using RNA polymerase II promoters using no additional processing elements (*e*.*g*. tRNA processing, ribozymes, or Cys4 cleavage sites) *Yarrowia lipolytica* was transformed with the constructs described in Example 3 and targeting efficiency at the Can1-1 target site (SEQ ID NO: 69) was monitored.

A uracil auxotroph of *Yarrowia lipolytica* ATCC20362 was transformed with 100ng of pZufCas9 (SEQ ID NO: 18), pRF303 (SEQ ID NO: 70), pRF617 (SEQ ID NO: 61), pRF616 (SEQ ID NO: 62), pRF619 (SEQ ID NO: 63), pRF618 (SEQ ID NO: 64), pRF623 (SEQ ID NO: 67), pRF621 (SEQ ID NO: 68), or no DNA using standard lithium acetate transformation techniques. Post transformation cells were plated on CM-ura medium solidified with 1.8% w/v Bacto agar (Teknova). Plates were incubated at 25°C for 48 hours. 32 colonies from each transformation were patched onto complete minimal medium lacking arginine and containing 60µg/ml L-canavanine. L-canavanine is toxic to cells with a functional *CAN1* gene which is an importer of arginine and L-canavanine to the cells. Cells containing a loss of function allele in the *CAN1* gene will be phenotypically resistant to the presence of L-canavanine in the medium and will form colonies on plates containing L-canavanine. Cells containing a wild-type copy of the *CAN1* gene will be unable to grow on medium containing L-canavanine. The mode of action of L-canavanine is well known (Rosenthal G.A., The Biological effects and mode of action of L-Canavanine, a structural analog of L-arginine, The quarterly review of biology, volume 52, 1977, 155-178).

Colonies from cells transformed with pZufCas9 (SEQ ID NO: 18) which expresses Cas9 but does not contain a gRNA expression cassette yield no Canavanine resistant colonies (Figure 4, Table 4).

**Table 4**

| **Frequency of *CAN1* loss of function mutations by various gRNA expression cassettes** | | | | | |
|---|---|---|---|---|---|
| **Construct** | **Promoter** | **VT domain** | **3' HDV domain** | **Terminator** | **Can^{R}/Total** |
| pZufCas9 | none | None | no | none | 0/32 |
| pRF303 | YL52 | Can1-1 | no | *SUP4* | 28/32 |
| pRF616 | *FBA1_{TSS}* | Can1-1 | yes | *ACT1* | 22/32 |
| pRF617 | *FBA1_{TSS}* | Can1-1 | no | *ACT1* | 24/32 |
| pRF618 | *FBA1_{UTR}* | Can1-1 | yes | *ACT1* | 32/32 |
| pRF619 | *FBA1_{UTR}* | Can1-1 | no | *ACT1* | 20/32 |
| pRF621 | *TEF1_{UTR}* | Can1-1 | yes | *ACT1* | 30/32 |
| pRF623 | *TEF1_{UTR}* | Can1-1 | no | *ACT1* | 27/32 |

Colonies from cells transformed with pRF303 (SEQ ID NO: 70) which contains a gRNA expression cassette driven by an RNA polymerase III promoter and an 5' HDV ribozyme for processing produces relatively pure mutant colonies (Figure 4) at a frequency of 88% (Table 3). All constructs containing the Pol II promoter gRNA expression cassette also produced colonies that contained Canavaine resistant cells with similar frequency (ca. 69% to 100%, Table 3). However, with the exception of pRF618 (SEQ ID NO: 64), pRF621 (SEQ ID NO: 68), and pRF623 (SEQ ID NO: 67) the colonies were mostly non-mutant cells as demonstrated by the weak patches on L-canavanine containing medium (Figure 4). Both *TEF1* and *FBA1* constructs are improved by the addition of an HDV domain 3' of the gRNA in the expression cassette suggesting that the Pol II terminator may leave sequences that inhibit Cas9/gRNA targeting. Additionally constructs containing the 5' UTR of the promoter function more efficiently than constructs where the gRNA is fused directly to the transcription start site (Figure 4), not affecting overall frequency (Table 4) but increasing the ratio of mutant:WT cells within a colony arising from a single transformed cell.

The data presented in this example demonstrates that gRNAs can be expressed from RNA polymerase II promoters with no additional processing elements as fusions with either the transcriptional start site or the end of the 5' untranslated region. The addition of a ribozyme between the gRNA and the terminator sequence improves targeting. The efficiency of these gRNAs is at least as good as incumbent expression systems using RNA polymerase III promoters and/or processing elements but opens a much larger pool of promoters for gRNA expression including the possibility of tissue and condition specific gRNA expression that is not possible with RNA polymerase III promoters.

## Claims

1. A recombinant DNA construct comprising:
i) a Polymerase II (Pol-II) promoter fused to its 5' UTR and operably linked to a polynucleotide encoding a single guide RNA comprising a variable targeting domain and a Cas endonuclease recognition domain;
ii) a 3' processing element operably linked to the polynucleotide; and, optionally,
iii) a terminator located 3' of said processing element;
wherein said guide RNA is capable of forming a guide RNA/Cas endonuclease complex, which complex can bind to and cleave a target site sequence in the genome of a eukaryote, and wherein said polynucleotide contains no 5' processing element.

2. The recombinant DNA construct of claim 1, wherein the 3' processing element is a ribozyme.

3. The recombinant DNA construct of claim 1 or 2, wherein the eukaryote is selected from the group comprising a microbe, a yeast, a non-conventional yeast, a fungus, a plant, a non-human animal, an insect and a nematode.

4. A non-human eukaryote selected from a microbe, a yeast, a non-conventional yeast, a fungus, and a plant, or a non-human eukaryotic cell, comprising the recombinant DNA construct of claim 1 or 2.

5. An expression vector comprising at least one recombinant DNA construct of claim 1 or 2.

6. The expression vector of claim 5, further comprising a polynucleotide encoding a Cas endonuclease, a polynucleotide modification template or a donor DNA.

7. A method for modifying a target site on a chromosome or episome in a non-conventional yeast, the method comprising providing to a non-conventional yeast at least a first recombinant DNA construct of claim 1 and a second recombinant DNA construct encoding a Cas endonuclease, wherein the Cas endonuclease introduces a single or double-strand break at said target site; and
wherein said method is not a method for the treatment of a human or animal body by therapy or surgery.

8. The method of claim 7, wherein the at least first recombinant DNA construct of claim 1 and the second recombinant DNA construct are located on the same polynucleotide or separate polynucleotides.

9. The method of claim 7 or 8, further comprising identifying at least one non-conventional yeast cell that has a modification at said target site, wherein the modification includes at least one deletion, addition or substitution of one or more nucleotides in said target site.

10. The method of claim 9, wherein said method further comprises identifying the mutation efficiency in said non-conventional yeast.

11. The method of claim 7 or 8 further comprising providing a donor DNA to said yeast, wherein said donor DNA comprises a polynucleotide of interest.

12. The method of claim 11, further comprising identifying at least one yeast cell comprising in its chromosome or episome the polynucleotide of interest integrated at said target site.

13. A method for editing a nucleotide sequence on a chromosome or episome in a non-conventional yeast, the method comprising providing to a non-conventional yeast a polynucleotide modification template DNA, a first recombinant DNA construct comprising a DNA sequence encoding a Cas endonuclease, and a second recombinant DNA construct of claim 1, wherein the Cas endonuclease introduces a single or double-strand break at a target site in the chromosome or episome of said yeast, wherein said polynucleotide modification template DNA comprises at least one nucleotide modification of said nucleotide sequence; and
wherein said method is not a method for the treatment of a human or animal body by therapy or surgery.

14. A method for silencing a nucleotide sequence on a chromosome or episome in a non-conventional yeast, the method comprising providing to a non-conventional yeast at least a first recombinant DNA construct comprising a DNA sequence encoding an inactivated Cas endonuclease, and at least a second recombinant DNA construct of claim 1, wherein the guide RNA molecule and the inactivated Cas endonuclease can form a complex that binds to said nucleotide sequence in the chromosome or episome of said yeast, thereby blocking transcription of said nucleotide sequence; and
wherein said method is not a method for the treatment of a human or animal body by therapy or surgery.

## Patentansprüche

1. Rekombinantes DNA-Konstrukt, umfassend:
i) einen Polymerase-II(Pol-II)-Promotor, der an seine 5'-UTR fusioniert ist und mit einem Polynukleotid funktionell verknüpft ist, das eine einzelne guideRNA codiert, die eine variable Targeting-Domäne und eine Cas-Endonuklease-Erkennungsdomäne umfasst;
ii) ein 3'-Prozessierungselement, das mit dem Polynukleotid funktionell verknüpft ist, und gegebenenfalls
iii) einen Terminator, der in 3'-Richtung von dem Prozessierungselement liegt;
wobei die guideRNA in der Lage ist, einen guideRNA/Cas-Endonuklease-Komplex zu bilden, wobei der Komplex an eine Zielstellensequenz im Genom eines Eukaryoten binden und diese spalten kann und wobei das Polynukleotid kein 5'-Prozessierungselement enthält.

2. Rekombinantes DNA-Konstrukt nach Anspruch 1, wobei es sich bei dem 3'-Prozessierungselement um ein Ribozym handelt.

3. Rekombinantes DNA-Konstrukt nach Anspruch 1 oder 2, wobei der Eukaryot aus der eine Mikrobe, eine Hefe, eine unkonventionelle Hefe, einen Pilz, eine Pflanze, ein nicht menschliches Tier, ein Insekt und einen Fadenwurm umfassenden Gruppe ausgewählt ist.

4. Nicht menschlicher Eukaryot ausgewählt aus einer Mikrobe, einer Hefe, einer unkonventionellen Hefe, einem Pilz und einer Pflanze oder nicht menschliche eukaryotische Zelle, umfassend das rekombinante DNA-Konstrukt nach Anspruch 1 oder 2.

5. Expressionsvektor, umfassend mindestens ein rekombinantes DNA-Konstrukt nach Anspruch 1 oder 2.

6. Expressionsvektor nach Anspruch 5, ferner umfassend ein Polynukleotid, das eine Cas-Endonuklease, eine Polynukleotidmodifikationsvorlage oder eine Spender-DNA codiert.

7. Verfahren zum Modifizieren einer Zielstelle auf einem Chromosom oder Episom in einer unkonventionellen Hefe, wobei das Verfahren Bereitstellen mindestens eines ersten rekombinanten DNA-Konstrukts nach Anspruch 1 und eines zweiten eine Cas-Endonuklease codierenden rekombinanten DNA-Konstrukts an eine unkonventionelle Hefe umfasst, wobei die Cas-Endonuklease einen Einzel- oder Doppelstrangbruch bei der Zielstelle einführt und wobei das Verfahren kein Verfahren zur Behandlung eines menschlichen oder tierischen Körpers durch Therapie oder Operation darstellt.

8. Verfahren nach Anspruch 7, wobei das mindestens erste rekombinante DNA-Konstrukt nach Anspruch 1 und das zweite rekombinante DNA-Konstrukt auf demselben Polynukleotid oder auf verschiedenen Polynukleotiden liegen.

9. Verfahren nach Anspruch 7 oder 8, ferner umfassend Identifizieren mindestens einer unkonventionellen Hefezelle, die eine Modifikation an der Zielstelle aufweist, wobei die Modifikation mindestens eine Deletion, Addition oder Substitution eines oder mehrerer Nukleotide innerhalb der Zielstelle enthält.

10. Verfahren nach Anspruch 9, wobei das Verfahren ferner Identifizieren der Mutationseffizienz in der unkonventionellen Hefe umfasst.

11. Verfahren nach Anspruch 7 oder 8, ferner umfassend Bereitstellen einer Spender-DNA an die Hefe, wobei die Spender-DNA ein Polynukleotid von Interesse umfasst.

12. Verfahren nach Anspruch 11, ferner umfassend Identifizieren mindestens einer Hefezelle, die das an der Zielstelle integrierte Polynukleotid von Interesse in seinem Chromosom oder Episom umfasst.

13. Verfahren zum Editing einer Nukleotidsequenz auf einem Chromosom oder Episom in einer unkonventionellen Hefe, wobei das Verfahren Bereitstellen einer Polynukleotidmodifikationsvorlagen-DNA, eines ersten rekombinanten DNA-Konstrukts, das eine eine Cas-Endonuklease codierende DNA-Sequenz umfasst, und eines zweiten rekombinanten DNA-Konstrukts nach Anspruch 1 an eine unkonventionelle Hefe umfasst, wobei die Cas-Endonuklease einen Einzel- oder Doppelstrangbruch an einer Zielstelle in dem Chromosom oder Episom der Hefe einführt, wobei die Polynukleotidmodifikationsvorlagen-DNA mindestens eine Nukleotidmodifikation der Nukleotidsequenz umfasst und
wobei das Verfahren kein Verfahren zur Behandlung eines menschlichen oder tierischen Körpers durch Therapie oder Operation darstellt.

14. Verfahren zum Silencing einer Nukleotidsequenz auf einem Chromosom oder Episom in einer unkonventionellen Hefe, wobei das Verfahren Bereitstellen mindestens eines ersten rekombinanten DNA-Konstrukts, das eine eine inaktivierte Cas-Endonuklease codierende DNA-Sequenz umfasst, und mindestens eines zweiten rekombinanten DNA-Konstrukts nach Anspruch 1 an eine unkonventionelle Hefe umfasst, wobei das guideRNA-Molekül und die inaktivierte Cas-Endonuklease einen Komplex bilden können, der an die Nukleotidsequenz in dem Chromosom oder Episom der Hefe bindet, wodurch die Transkription der Nukleotidsequenz blockiert wird, und
wobei das Verfahren kein Verfahren zur Behandlung eines menschlichen oder tierischen Körpers durch Therapie oder Operation darstellt.

## Revendications

1. Construction d'ADN recombinant comprenant :
i) un promoteur de polymérase II (Pol-II) fusionné à sa région 5' UTR et lié fonctionnellement à un polynucléotide codant pour un ARN guide unique comprenant un domaine de ciblage variable et un domaine de reconnaissance de l'endonucléase Cas ;
ii) un élément de traitement 3' lié fonctionnellement au polynucléotide ; et, facultativement,
iii) un terminateur situé en 3' dudit élément de traitement ;
dans laquelle ledit ARN guide peut former un complexe ARN guide/endonucléase Cas, ledit complexe pouvant se lier à une séquence de site cible dans le génome d'un eucaryote et cliver celle-ci, et ledit polynucléotide ne contenant pas d'élément de traitement 5'.

2. Construction d'ADN recombinant selon la revendication 1, dans laquelle l'élément de traitement 3' est un ribozyme.

3. Construction d'ADN recombinant selon la revendication 1 ou 2, l'eucaryote étant choisi parmi un microbe, une levure, une levure non conventionnelle, un champignon, une plante, un animal non humain, un insecte et un nématode.

4. Eucaryote non humain choisi parmi un microbe, une levure, une levure non conventionnelle, un champignon et une plante, ou une cellule eucaryote non humaine, comprenant la construction d'ADN recombinant selon la revendication 1 ou 2.

5. Vecteur d'expression comprenant au moins une construction d'ADN recombinant selon la revendication 1 ou 2.

6. Vecteur d'expression selon la revendication 5, comprenant en outre un polynucléotide codant pour une endonucléase Cas, une matrice de modification de polynucléotide ou un ADN donneur.

7. Procédé de modification d'un site cible sur un chromosome ou un épisome chez une levure non conventionnelle, le procédé comprenant la fourniture à une levure non conventionnelle d'au moins une première construction d'ADN recombinant selon la revendication 1 et d'une deuxième construction d'ADN recombinant codant pour une endonucléase Cas, l'endonucléase Cas introduisant une cassure simple ou double brin au niveau dudit site cible ; et
ledit procédé ne constituant pas une méthode de traitement thérapeutique ou chirurgical du corps humain ou animal.

8. Procédé selon la revendication 7, dans lequel la ou les premières constructions d'ADN recombinant selon la revendication 1 et la deuxième construction d'ADN recombinant sont situées sur le même polynucléotide ou sur des polynucléotides distincts.

9. Procédé selon la revendication 7 ou 8, comprenant en outre l'identification d'au moins une cellule de levure non conventionnelle qui présente une modification au niveau dudit site cible, la modification comprenant au moins une délétion, une addition ou une substitution d'un ou plusieurs nucléotides dans ledit site cible.

10. Procédé selon la revendication 9, ledit procédé comprenant en outre l'identification de l'efficacité de mutation dans ladite levure non conventionnelle.

11. Procédé selon la revendication 7 ou 8, comprenant en outre la fourniture d'un ADN donneur à ladite levure, ledit ADN donneur comprenant un polynucléotide d'intérêt.

12. Procédé selon la revendication 11, comprenant en outre l'identification d'au moins une cellule de levure comprenant, dans son chromosome ou son épisome, le polynucléotide d'intérêt intégré au niveau dudit site cible.

13. Procédé d'édition d'une séquence nucléotidique sur un chromosome ou un épisome chez une levure non conventionnelle, le procédé comprenant la fourniture à une levure non conventionnelle d'un ADN matrice de modification de polynucléotide, d'une première construction d'ADN recombinant comprenant une séquence d'ADN codant pour une endonucléase Cas, et d'une deuxième construction d'ADN recombinant selon la revendication 1, l'endonucléase Cas induisant une cassure simple ou double brin au niveau d'un site cible dans le chromosome ou l'épisome de ladite levure, ledit ADN matrice de modification de polynucléotide comprenant au moins une modification nucléotidique de ladite séquence nucléotidique ; et
ledit procédé ne constituant pas une méthode de traitement thérapeutique ou chirurgical du corps humain ou animal.

14. Procédé de silençage d'une séquence nucléotidique sur un chromosome ou un épisome chez une levure non conventionnelle, le procédé comprenant la fourniture à une levure non conventionnelle d'au moins une première construction d'ADN recombinant comprenant une séquence d'ADN codant pour une endonucléase Cas inactivée, et d'au moins une deuxième construction d'ADN recombinant selon la revendication 1, la molécule d'ARN guide et l'endonucléase Cas inactivée pouvant former un complexe qui se lie à ladite séquence nucléotidique dans le chromosome ou l'épisome de ladite levure, bloquant ainsi la transcription de ladite séquence nucléotidique ; et ledit procédé ne constituant pas une méthode de traitement thérapeutique ou chirurgical du corps humain ou animal.
